(19) Europäisches Patentamt
European Patent Office
Office européen des brevets

(11) Veröffentlichungsnummer: **0 236 920 B1**

(12) # EUROPÄISCHE PATENTSCHRIFT

(45) Veröffentlichungstag der Patentschrift: **21.10.92**

(21) Anmeldenummer: **87103030.0**

(22) Anmeldetag: **04.03.87**

Teilanmeldung 91120740.5 eingereicht am 04/03/87.

(51) Int. Cl.[5]: **C12N 15/20**, A61K 45/00, C12N 1/20, C12N 5/12, C07K 15/26, C07K 3/18, C07K 17/00, //(C12N1/20, C12R1:19)

(54) **Hybridinterferone, deren Verwendung als Arzneimittel und als Zwischenprodukte zur Herstellung von Antikörpern und deren Verwendung sowie Verfahren zu ihrer Herstellung.**

(30) Priorität: **10.03.86 DE 3607835**

(43) Veröffentlichungstag der Anmeldung: **16.09.87 Patentblatt 87/38**

(45) Bekanntmachung des Hinweises auf die Patenterteilung: **21.10.92 Patentblatt 92/43**

(84) Benannte Vertragsstaaten: **AT BE CH DE ES FR GB GR IT LI LU NL SE**

(56) Entgegenhaltungen:
**EP-A- 0 051 873**
**EP-A- 0 170 204**
**WO-A-80/02229**
**WO-A-83/02461**

(73) Patentinhaber: **BOEHRINGER INGELHEIM INTERNATIONAL GmbH**
**Postfach 20**
**W-6507 Ingelheim am Rhein(DE)**

(72) Erfinder: **Hauptmann, Rudolf, Dr.**
**Döllachstrasse 22**
**A-2483 Ebreichsdorf(AT)**
Erfinder: **Swetly, Peter, Prof. Dr.**
**Hietzinger Hauptstrasse 40 B,**
**A-1130 Wien(AT)**
Erfinder: **Meindl, Peter, Dr.**
**Hockegasse 63/1,**
**A-1180 Wien(AT)**
Erfinder: **Günther, Adolf, Mag. Dr.**
**Johannagasse 20/7,**
**A-1050 Wien(AT)**
Erfinder: **Falkner, Edgar, Dr.**
**Strohberggasse 9,**
**A-1120 Wien(AT)**

**EP 0 236 920 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**JOURNAL OF BIOLOGICAL CHEMISTRY, vol. 257, no. 19, 10. Oktober 1982, Seiten 11497-11502, Bethesda, NJ, US; E. REHBERG et al.: "Specific molecular activities of recombinant and hybrid leukocyte interferons"**

Erfinder: **Bodo, Gerhard, Prof. Dr.**
**Belghofergasse 27/5,**
**A-1120 Wien(AT)**
Erfinder: **Maurer-Fogy, Ingrid, Dr.**
**Lindauergasse 35,**
**A-1238 Wien(AT)**

**Beschreibung**

In Nucleic Acids Res. 13, 4739-4749 (1985) (siehe auch EP-A-0.170.204) wird eine neue Klasse von Typ I Interferonen beschrieben, welche als omega-Interferone bezeichnet werden, die für sie kodierende DNA-Sequenzen, Plasmide, die diese DNA-Sequenzen enthalten, und die neuen Interferone produzierende Organismen.

Bei der Herstellung von größeren Versuchsmengen der neuen omega-Interferone mittels den in den oben erwähnten Publikationen beschriebenen Expressionsplasmiden, z.B. mit pRHW11 oder pRHW12 jeweils transformiert in E. coli HB101, zeigte es sich jedoch, daß es wünschenswert wäre, die Expression der neuen omega-Interferone zu steigern und gleichzeitig die erforderliche nachfolgende Reinigung der exprimierten omega-Interferone zu verbessern.

Überraschenderweise wurde nun gefunden, daß die vorstehend erwähnten Unzulänglichkeiten mit Hilfe der neuen BglII-Hybridinterferone der allgemeinen Formel

```
R1 - Gln Ile Phe - R2
     CAG ATC TTC              (I)
      BglII
```

in der

BglII die gemeinsame Bg1II-Restriktionsstelle der $\alpha$1-, $\alpha$2-und omega-Interferone,

$R_1$ die Peptidsequenz eines $\alpha$1- oder $\alpha$2-Interferons, die durch die DNA-Sequenz dieser Interferone vor der BglII-Schnittstelle kodiert wird, und $R_2$ die Peptidsequenz eines omega-Interferons, die durch die DNA-Sequenz dieses Interferons nach der BglII-Schnittstelle kodiert wird, oder

$R_1$ die Peptidsequenz eines omega-Interferons, die durch die DNA-Sequenz dieses Interferons vor der BglII-Schnittstelle kodiert wird, und $R_2$ die Peptidsequenz eines $\alpha$l- oder $\alpha$2-Interferons, die durch die DNA-Sequenz dieser Interferone nach der BglII-Schnittstelle kodiert wird, bedeuten, deren N-terminale Met- oder N-Formyl-Met-Derivate und, falls die Peptidsequenz des Hybridinterferons eine Glykosylierungsstelle enthält, deren N-glykosylierte Derivate, sowie durch die Konstruktion eines neuen Plasmids, mit dem die Expression der omega-Interferone verbessert werden konnte, behoben werden.

Die neuen Hybridinterferone, deren N-terminale Met- oder N-Formyl-Met-Derivate und, falls die Peptidsequenz des Hybridinterferons eine Glykosylierungsstelle enthält, deren N-glykosylierte Derivate weisen im Gegensatz zu der Nachveröffentlichung in Journal of Interferon Research 9, 97-114 (1989) eine größere spezifische antivirale Aktivität als die entsprechenden Basisinterferone auf, diese sind jedoch insbesondere zur Herstellung von neuen monoklonalen Antikörpern, welche zur Reinigung von $\alpha$- und omega-Interferonen geeignet sind, verwendbar.

Gegenstand der vorliegenden Erfindung sind somit die neuen BglII-Hybridinterferone der vorstehenden Formel I, deren N-terminale Met- oder N-Formyl-Met-Derivate und, falls die Peptidsequenz des Hybridinterferons eine Glykosylierungsstelle enthält, deren N-glykosylierte Derivate, deren Verwendung als Arzneimittel und als Zwischenprodukte, die für diese Hybridinterferone kodierende DNA-Sequenzen und Verfahren zu ihrer Herstellung,

neue Plasmide zur Verbesserung der Expression der omega-Interferone und neue Zwischenplasmide zur Herstellung der neuen Plasmide sowie Verfahren zu ihrer Herstellung.

Erfindungsgemäß wird zur Herstellung der vorstehend genannten Gegenstände wie folgt verfahren:

Die neuen BglII-Hybridinterferone der vorliegenden Erfindung sind aus einem Teil eines $\alpha$-Interferons, vorzugsweise aus einem Teil eines $\alpha$1- oder $\alpha$2-Interferons, z.B. des IFN-$\alpha$2(Arg) (siehe EP-A-0.095.702), und einem Teil eines omega-Interferons, vorzugsweise aus einem Teil des omega1(Glu)- oder omega1(Gly)-Interferons (siehe EP-A-0.170.204), aufgebaut. Hierbei erfolgt beispielsweise beim IFN-$\alpha$2(Arg) und IFN-omega1 die Verknüpfung der für die entsprechenden Teile kodierende DNA-Sequenzen über die BglII-Schnittstelle, die sich in den Positionen 191-196 in beiden Genen befindet. Hierbei muß eine gegebenenfalls vorhandende Lücke in einem Peptid mit den Aminosäuren 1 bis 66 eines $\alpha$-Interferons, z.B. die Lücke für die Aminosäure Nr. 45 im IFN-$\alpha$2(Arg), mitgezählt werden, damit die Sequenz beider Gene miteinander verglichen werden kann, wie aus der nachfolgenden Abbildung hervorgeht (einschließlich des N-terminalen Met-Restes, welcher nach der bakteriellen Proteinsynthese meistens wieder abgespalten wird):

```
                    5                   10                  15
Met Cys Asp Leu Pro Gln Thr His Ser Leu Gly Ser Arg Arg Thr
ATG TGT GAT CTG CCT CAA ACC CAC AGC CTG GGT AGC AGG AGG ACC   45
Met Cys Asp Leu Pro Gln Asn His Gly Leu Leu Ser Arg Asn Thr
ATG TGT GAT CTG CCT CAG AAC CAT GGC CTA CTT AGC AGG AAC ACC   45

                    20                  15                  30
Leu Met Leu Leu Ala Gln Met Arg Arg Ile Ser Leu Phe Ser Cys
TTG ATG CTC CTG GCA CAG ATG AGG AGA ATC TCT CTT TTC TCC TGC   90
Leu Val Leu Leu His Gln Met Arg Arg Ile Ser Pro Phe Leu Cys
TTG GTG CTT CTG CAC CAA ATG AGG AGA ATC TCC CCT TTC TTG TGT   90

                    35                  40                  45
Leu Lys Asp Arg Arg Asp Phe Gly Phe Pro Gln Glu Glu Phe ...
TTG AAG GAC AGA CGT GAC TTT GGA TTT CCC CAG GAG GAG TTT ... 135
Leu Lys Asp Arg Arg Asp Phe Arg Phe Pro Gln Glu Met Val Lys
CTC AAG GAC AGA AGA GAC TTC AGG TTC CCC CAG GAG ATG GTA AAA 135

                    50                  55                  60
Gly Asn Gln Phe Gln Lys Ala Glu Thr Ile Pro Val Leu His Glu
GGC AAC CAG TTC CAA AAG GCT GAA ACC ATC CCT GTC CTC CAT GAG 180
Gly Ser Gln Leu Gln Lys Ala His Val Met Ser Val Leu His Glu
GGG AGC CAG TTG CAG AAG GCC CAT GTC ATG TCT GTC CTC CAT GAG 180

                    65                  70                  75
Met Ile Gln Gln Ile Phe Asn Leu Phe Ser Thr Lys Asp Ser Ser
ATG ATC CAG CAG ATC TTC AAT CTC TTC AGC ACA AAG GAC TCA TCT 225
Met Leu Gln Gln Ile Phe Ser Leu Phe His Thr Glu Arg Ser Ser
ATG CTG CAG CAG ATC TTC AGC CTC TTC CAC ACA GAG CGC TCC TCT 225
```

```
                    80                  85                  90
Ala Ala Trp Asp Glu Thr Leu Leu Asp Lys Phe Tyr Thr Glu Leu
GCT GCT TGG GAT GAG ACC CTC CTA GAC AAA TTC TAC ACT GAA CTC 270
Ala Ala Trp Asn Met Thr Leu Leu Asp Gln Leu His Thr Gly Leu
GCT GCC TGG AAC ATG ACC CTC CTA GAC CAA CTC CAC ACT GGA CTT 270

                    95                 100                 105
Tyr Gln Gln Leu Asn Asp Leu Glu Ala Cys Val Ile Gln Gly Val
TAC CAG CAG CTG AAT GAC CTG GAA GCC TGT GTG ATA CAG GGG GTG 315
His Gln Gln Leu Gln His Leu Glu Thr Cys Leu Leu Gln Val Val
CAT CAG CAA CTG CAA CAC CTG GAG ACC TGC TTG CTG CAG GTA GTG 315

                   110                 115                 120
Gly Val Thr Glu Thr Pro Leu Met Lys Glu Asp Ser Ile Leu Ala
GGG GTG ACA GAG ACT CCC CTG ATG AAG GAG GAC TCC ATT CTG GCT 360
Gly Glu Gly Glu Ser Ala Gly Ala Ile Ser Ser Pro Ala Leu Thr
GGA GAA GGA GAA TCT GCT GGG GCA ATT AGC AGC CCT GCA CTG ACC 360

                   125                 130                 135
Val Arg Lys Tyr Phe Gln Arg Ile Thr Leu Tyr Leu Lys Glu Lys
GTG AGG AAA TAC TTC CAA AGA ATC ACT CTC TAT CTG AAA GAG AAG 405
Leu Arg Arg Tyr Phe Gln Gly Ile Arg Val Tyr Leu Lys Glu Lys
TTG AGG AGG TAC TTC CAG GGA ATC CGT GTC TAC CTG AAA GAG AAG 405

                   140                 145                 150
Lys Tyr Ser Pro Cys Ala Trp Glu Val Val Arg Ala Glu Ile Met
AAA TAC AGC CCT TGT GCC TGG GAG GTT GTC AGA GCA GAA ATC ATG 450
Lys Tyr Ser Asp Cys Ala Trp Glu Val Val Arg Met Glu Ile Met
AAA TAC AGC GAC TGT GCC TGG GAA GTT GTC AGA ATG GAA ATC ATG 450

                   155                 160                 165
Arg Ser Phe Ser Leu Ser Thr Asn Leu Gln Glu Ser Leu Arg Ser
AGA TCT TTT TCT TTG TCA ACA AAC TTG CAA GAA AGT TTA AGA AGT 495
Lys Ser Leu Phe Leu Ser Thr Asn Met Gln Glu Arg Leu Arg Ser
AAA TCC TTG TTC TTA TCA ACA AAC ATG CAA GAA AGA CTG AGA AGT 495
```

```
                    170
Lys Glu
AAG GAA TGA                                                504
Lys Asp Arg Asp Leu Gly Ser Ser
AAA GAT AGA GAC CTG GGC TCA TCT TGA                        522
```

In der obigen Abbildung stellt in jeder Doppelreihe die erste Reihe die entsprechenden Sequenzen des IFN-α2(Arg) und die zweite Reihe die des omega1-Interferons dar, hierbei befindet sich in den Nukleotidpositionen 191-196 die gemeinsame BglII-Schnittstelle für beide Gene - das IFN-α2(Arg)-Gen weist an der Nukleotidposition 451-456 eine zweite BglII-Schnittstelle auf.

Ferner enthalten die nachfolgenden nicht maßstabgerechten Darstellungen der einzelnen Plasmide nur die wesentlichen Sequenzen und Restriktionsenzymerkennungsstellen. Hierbei wurden folgende Abkürzungen benutzt:

Restriktionsenzymerkennungssequenzen:

B: BamHI, Bg: BglII, Bgl: 1.BglII-Stelle im IFN-α2(Arg)-Gen, Bg2: 2.BglII-Stelle im IFN-α2(Arg)-Gen, E: EcoRI, H: HindIII, N: NcoI, P: PstI, S: SphI

p/o: Tryptophan Promotor/Operator (Serratia marcescens) mit anschließender Shine-Dalgarno Sequenz (ribosomale Bindungsstelle)

- par: partition Lokus aus dem Plasmid pPM31
- ori: Replikationsursprung
- $Ap^r$: Ampicillin Resistenzgen
- $Tc^r$: Tetracyclin Resistenzgen
- $Tc^s$: Tetracyclin sensitiv
- kb: 1000 Basenpaare

Desweiteren sind in der vorliegenden Anmeldung folgende Figuren enthalten:

- Fig. 1: Konstruktionsschema für parpATER33
- Fig. 2: Konstruktionsschema für pRHW14
- Fig. 3: Autoradiogramm von $^{35}S$ markierten Proteinen aus Maxizellen (E.coli CSR 603)
- Fig. 4: Konstruktionsschema für pRH72
- Fig. 5: Konstruktionsschema für pRH78r und pRH78f
- Fig. 6: MONO-S Chromatogramm von IFN-omega1/α2(BglII) AUFS = Absorption units full scale
- Fig. 7: Gelpermeations-HPLC von IFN-omega1/α2(BglII)

Die neuen BglII-Hybridinterferone und deren N-glykosylierte Derivate bestehen also entweder aus den Aminosäuren 1-66 eines α1- oder α2-Interferons, vorzugsweise aus den Aminosäuren 1-65 des IFN-α2(Arg), und den Aminosäuren 67 bis 173 eines omega1-Interferons oder aus den Aminosäuren 1 bis 66 des omega1-Interferons und den Aminosäuren 67 bis 167 eines α1- oder α2-Interferons, vorzugsweise aus den Aminosäuren 66 bis 166 des IFN-α2(Arg), wobei der N-terminale Met-Rest nach der bakteriellen Proteinsynthese meistens wieder abgespalten wird.

Überraschenderweise werden die neuen Hybridinterferone von Anti-IFN-α-Antikörpern erkannt. Dadurch wird die Reinigung der neuen Hybridinterferone mittels literaturbekannter Anti-IFN-α-Antikörpern (siehe beispielsweise EP-A-0.119.476) möglich. Mit einem so erhaltenen reinen neuen Hybridinterferon kann dann durch Immunisierung eines Versuchstieres wie BALB/c-Mäusen die Bildung des entsprechenden Antikörpers induziert werden. Diese neuen Antikörper erkennen nicht nur die neuen Hybridinterferone, sondern überraschenderweise auch die einzelnen Bausteine der Hybridinterferone, z.B. ein omega-Interferon.

Die Ausgangsbasis zur Konstruktion der entsprechenden Hybridinterferon-Plasmide ist somit die gemeinsame BglII-Restriktionsstelle in den Positionen 191-196 eines α1-, α2- und omega1-Interferongens, wie dies bereits vorstehend erwähnt wurde, wobei die Lücke für das Codon Nr. 45 im IFN-α2(Arg)-Gen mitgezählt wurde, sowie zur Isolierung des entsprechenden, erforderlichen Gens die für ein α1-, α2-Interferon bzw. für ein omega1-Interferon kodierenden Plasmide.

Hierzu wird nun zuerst erfindungsgemäß ein die omega1-Interferon-Expression verbesserndes neues Plasmid hergestellt. Hierbei dient als Ausgangsplasmid das literaturbekannte und käuflich erhältliche Plasmid pAT153 (Fa. Amersham, siehe auch A.J. Twigg et al. in Nature 283, 216-218 (1980)), beispielsweise das für IFN-α2(Arg) kodierende Plasmid parpER33 (siehe EP-A-0.115.613) und ein für ein omega1-

Interferon der Formel

```
                    5                   10                  15
Cys Asp Leu Pro Gln Asn His Gly Leu Leu Ser Arg Asn Thr Leu

                    20                  25                  30
Val Leu Leu His Gln Met Arg Arg Ile Ser Pro Phe Leu Cys Leu

                    35                  40                  45
Lys Asp Arg Arg Asp Phe Arg Phe Pro Gln Glu Met Val Lys Gly

                    50                  55                  60
Ser Gln Leu Gln Lys Ala His Val Met Ser Val Leu His Glu Met

                    65                  70                  75
Leu Gln Gln Ile Phe Ser Leu Phe His Thr Glu Arg Ser Ser Ala

                    80                  85                  90
Ala Trp Asn Met Thr Leu Leu Asp Gln Leu His Thr Gly Leu His

                    95                  100                 105
Gln Gln Leu Gln His Leu Glu Thr Cys Leu Leu Gln Val Val Gly

                    110                 115                 120
Glu Gly Glu Ser Ala -X- Ala Ile Ser Ser Pro Ala Leu Thr Leu

                    125                 130                 135
Arg Arg Tyr Phe Gln Gly Ile Arg Val Tyr Leu Lys Glu Lys Lys

                    140                 145                 150
Tyr Ser Asp Cys Ala Trp Glu Val Val Arg Met Glu Ile Met Lys

                    155                 160                 165
Ser Leu Phe Leu Ser Thr Asn Met Gln Glu Arg Leu Arg Ser Lys

                    170
Asp Arg Asp Leu Gly Ser Ser
```

in der
X in Position 111 für Glu oder Gly steht,
kodierendes Plasmid wie das Plasmid pRHW11 oder 12 (siehe EP-A-0.170.204):
Das Plasmid parpER33, welches den par-Lokus mit der Sequenz der Formel

```
GAATTCCGAC AGTAAGACGG GTAAGCCTGT TGATGATACC GCTGCCTTAC    50
TGGGTGCATT AGCCAGTCTG AATGACCTGT CACGGGATAA TCCGAAGTGG   100
TCAGACTGGA AAATCAGAGG GCAGGAACTG CTGAACAGCA AAAAGTCAGA   150
TAGCACCACA TAGCAGACCC GCCATAAAAC GCCCTGAGAA ZCCGTGACGG   200
GCTTTTCTTG TATTATGGGT AGTTTCCTTG CATGAATCCA TAAAAGGCGC   250
CTGTAGTGCC ATTTACCCCC ATTCACTGCC AGAGCCGTGA GCGCAGCGAA   300
CTGAATGTCA CGAAAAAGAC AWCGACTCAG GTGCCTGATG GTCGGAGACA   350
AAAGGAATAT TCAGCGATTT GCCCGAGGAA TTC                     383
```

in der
Z das Nukleotid G oder C und
W das Nukleotid G oder kein Nukleotid bedeuten, sowie die für die Expression erforderlichen Replikon- und Kontrollsequenzen des Plasmids pER103 (siehe Anspruch 9) enthält, wird mit BamHI und PstI geschnitten. Die dabei entstehenden beiden Fragmente wurden mittels Gelelektrophorese, z.B. mit 1%igem Agarosegel, aufgetrennt und das kleinere Fragment, welches das IFN-$\alpha$2(Arg)-Gen enthält, wurde mittels Elektroelution isoliert. Die so erhaltene DNA wurde anschließend durch Zusatz von Äthanol ausgefällt, abzentrifugiert und in einem geeigneten Puffer wie TE-Puffer, z.B. in 10 mMol Tris(hydroxymethyl)aminomethan(Tris), pH = 8,0, und 1 mMol Äthylendinitrilotetraessigsäure-dinatriumsalz (EDTA)) aufgenommen.

Das Plasmid pAT153 wird ebenfalls mit BamHI und PstI geschnitten. Die dabei entstehenden beiden Fragmente wurden mittels Gelelektrophorese, z.B. mit 1%igem Agarosegel, aufgetrennt, und das größere Fragment, welches den Replikationsursprung enthält, isoliert. Das so erhaltene größere Fragment wird anschließend mit dem aus dem Plasmid parpER33 erhaltenen kleineren Fragment in Gegenwart einer Ligase, z.B. $T_4$ DNA-Ligase, ligiert. Zur Replikation der entstehenden Plasmide werden Bakterien, vorzugsweise E.coli vom Stamm HB101 (Genotyp $F^-$ , hsdS20($r^-$, $m^-$), recA13, ara-14, proA2, lacY1, galK2, rpsL20 ($Sm^r$), xyl-5, mtl-1, supE44, $lambda^-$), mit einer $CaCl_2$-Lösung gewaschen, und die so erhaltenen, kompetenten E.coli HB 101 mit der Ligasereaktionsmischung vermischt, und nach Inkubation bei 0 °C wird die Plasmid- DNA durch Hitzeschock bei 42 °C für 2 Minuten von den Bakterien aufgenommen. Anschließend werden die transformierten Bakterien auf Ampicillin-haltigen LB-Agar (LB-Medium + 15 g/l Agar) plattiert. Auf diesem Agar können nur E. coli HB101-Bakterien wachsen, die ein rekombinantes Vektormolekül aufgenommen haben. Nach Inkubation bei 37 °C wurden 12 der entstandenen Kolonien ausgewählt und von diesen nach der Methode von Birnboim et al. (siehe Nucl. Acids Res. 7, 1513 (1979)) die Plasmide isoliert. Durch Restriktionsenzymdoppelverdauung der ausgewählten Plasmide mit PstI-BamHI, PstI-PvuII oder EcoRI-BamHI und anschließender Gelelektrophorese der erhaltenen Fragmente wurde die Richtigkeit der Konstruktion dieser Plasmide bestätigt. Ein so erhaltenes Plasmid wurde ausgewählt und mit parpATER33 (Konstruktionsschema: siehe Figur 1) bezeichnet. Dieses Plasmid zeigt transformiert in E. coli HB101 den Phänotyp $Ap^r$ (Ampicillin-Resistenz), $Tc^r$ (Tetracyclin-Resistenz).

Das so erhaltene Plasmid parpATER33 mit der Restriktionskarte

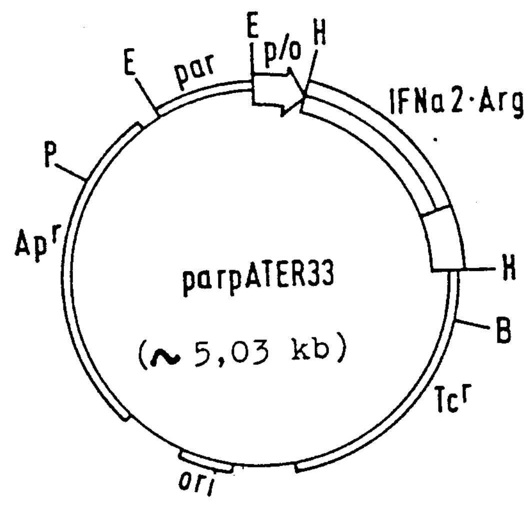


wird zur Herstellung eines die Expression von omega1-Interferon verbessernden Plasmids (Konstruktionsschema: siehe Figur 2) mit HindIII und BamHI geschnitten. Die dabei entstandenden drei Fragmente werden mittels Gelelektrophorese, z.B. mit 1%igem Agarosegel, aufgetrennt und das größte, ca. 3750 Basenpaare (bp) lange Fragment (Fragment a), welches den Tryptophan-Promotor/Operator (Serratia marcescens), den Replikationsursprung und das $Ap^r$ -Gen trägt, isoliert und mit der für ein omega1-Interferon kodierenden DNA ligiert. Diese DNA erhält man durch Schneiden eines für omega1-Interferon kodierenden Plasmid, vorzugsweise durch Schneiden des Plasmids pRHW11 bzw. pRHW12 mit BamHI und HindIII und anschließendem Auftrennen der beiden so erhaltenen Fragmente mittels Gelelektrophorese, wobei das gewünschte Gen in dem kleineren, ca. 800 bp langen Fragment enthalten ist. Zur Replikation der entstandenen Plasmide werden Bakterien, vorzugsweise E. coli HB101, mit einer $CaCl_2$-Lösung gewaschen und die so erhaltenen, kompetenten E. coli HB101 mit der Ligasereaktionsmischung vermischt, und nach Inkubation bei 0°C wird die Plasmid-DNA durch Hitzeschock bei 42°C für 2 Minuten von den Bakterien aufgenommen. Anschließend werden die transformierten Bakterien auf Ampicillin-haltigen LB-Agar plattiert. Auf diesem Agar können nur E. coli HB101-Bakterien wachsen, die ein rekombinantes Vektormolekül aufgenommen haben. Nach Inkubation bei 37°C werden 6 der entstandenen Kolonien ausgewählt, und von diesen nach der Methode von Birnboim et al. (siehe Nucl. Acids Res. 7, 1513 (1979)) die Plasmide isoliert. Durch Restriktionsenzymverdauung der ausgewählten Plasmide mit EcoRI, HindIII, NcoI und PstI und anschließender Gelelektrophorese wurde die Richtigkeit der Konstruktion dieser Plasmide bestätigt. Ein so erhaltenes Plasmid mit der Restriktionskarte

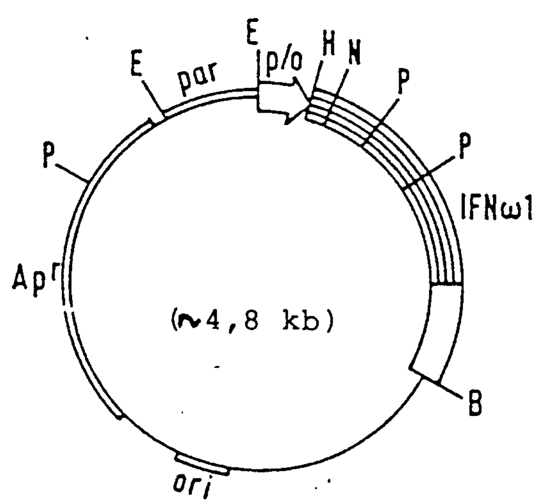


, wobei IFN ω 1 die für IFN-omega1(Gly) oder IFN-omega1(Glu) kodierende DNA-Sequenz darstellt, wurde ausgewählt, und mit pRHW14 (siehe Figur 2) bezeichnet, wenn als Ausgangsplasmid das Plasmid pRHW12, welches für omega1(Gly)-Interferon kodiert, verwendet wird. Dieses Plasmid zeigt transformiert in E. coli

HB101 den Phänotyp $Ap^r$ und $Tc^s$.

Bei Verwendung von pRHW11 als Ausgangsplasmid erhält man analog das Plasmid pRHW13, welches für omega1(Glu)-Interferon kodiert.

Das so erhaltene Plasmid pRHW14 weist eine doppelt so große Expressionsrate für das omega1(Gly)-Interferon auf als das bisher bekannte Plasmid pRHW12 (siehe EP-A-0.170.204) wie im Maxizell-System (siehe J. Bacteriol. 137, 692-693 (1979)) nachgewiesen werden kann. Hierzu wird E. coli CSR603 (Genotyp $F^-$, thr-1, leuB6, proA2, phr-1, recA1, argE3, thi-1, uvrA6, ara-14, lacY1, galK2, xyl-5, mtl-1, gyrA98 (nalA98), rpsL31, tsx-33, $lambda^-$, supE44), welcher defizient im Reparatursystem für UV-induzierte Schäden ist, mit den Plasmiden pRHW12 und pRHW14 transformiert. Wird die Strahlendosis so gewählt, daß zwar das Bakterienchromosom oft, die Plasmide aber kaum geschädigt werden, so erfolgt Transkription und in der Folge Translation vorwiegend nur von Plasmid-kodierten Genen. Enthält das Medium $^{35}S$-Methionin, so werden hauptsächlich Plasmid-Genprodukte markiert, die nach Auftrennen auf einem Acrylamidgel unter Verwendung einer Verstärkerfolie direkt mit Hilfe eines Röntgenfilms registriert werden können (siehe Figur 3). Das Ampicillin-Resistenzgenprodukt ($\beta$-Lactamase, bla) wird in beiden Fällen gleich stark markiert. Im Falle des pRHW14 ist jedoch das omega1(Gly)-Interferon doppelt so stark markiert wie beim pRHW12.

Zur Herstellung eines für ein Hybridinterferon der Formel I kodierenden Plasmids, das vor der gemeinsamen BglII-Schnittstelle die für $\alpha$1- oder $\alpha$2-Interferon kodierende DNA-Sequenzen enthält (Konstruktionsschema: siehe Figur 4), wird beispielsweise das Plasmid parpATER33 und ein für ein omega1-Interferon kodierendes Plasmid wie das Plasmid pRHW14 jeweils mit BglII und SphI geschnitten. Die hierbei entstandenen Fragmente wurden mittels Gelelektrophorese, z.B. mit 1%igem Agarosegel, aufgetrennt, eluiert und mittels Äthanol-Präzipitation gereinigt. Nach Lösen der Fragmente in einem geeigneten Puffer, z.B. in TE-Puffer, wurde das aus dem Plasmid parpATER33 erhaltene, große Fragment mit dem aus dem Plasmid pRHW14 erhaltenen, kleineren Fragment in Gegenwart einer Ligase, z.B. $T_4$ DNA-Ligase, ligiert. Zur Replikation der entstandenen Plasmide wurden Bakterien, vorzugsweise E.coli vom Stamm HB101 (Genotyp $F^-$, hsdS20($r^-$, $m^-$), recA13, ara-14, proA2, lacY1, galK2, rpsL20 ($Sm^r$), xyl-5, mtl-1, supE44, $lambda^-$), mit einer $CaCl_2$-Lösung gewaschen, und die so erhaltenen kompetenten E.coli HB 101 mit der Ligasereaktionsmischung vermischt, und nach Inkubation bei 0°C wird die Plasmid-DNA durch Hitzeschock bei 42°C für 2 Minuten von den Bakterien aufgenommen. Anschließend werden die transformierten Bakterien auf Ampicillin-haltigen LB-Agar (LB-Medium + 15 g/l Agar) plattiert. Auf diesem Agar können nur E. coli HB101-Bakterien wachsen, die ein rekombinantes Vektormolekül aufgenommen haben. Nach Inkubation bei 37°C wurden mehrere der entstandenen Kolonien ausgewählt und von diesen nach der Methode von Birnboim et al. (siehe Nucl. Acids Res. 7, 1513 (1979)) die Plasmide isoliert. Durch Restriktionsenzymdoppelverdauung der ausgewählten Plasmide mit BglII und SphI und anschließender Gelelektrophorese der erhaltenen Fragmente wurde die Richtigkeit der Konstruktion dieser Plasmide bestätigt. Ein so erhaltenes Plasmid wurde ausgewählt und mit pRH72 bezeichnet. Dieses Plasmid mit der Restriktionskarte

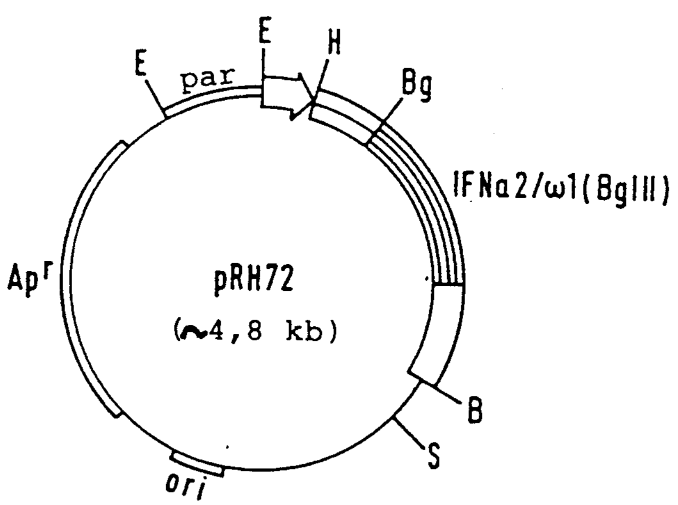


zeigt transformiert in E. coli HB101 den Phänotyp $Ap^r$ und $Tc^s$ und kodiert für das IFN-$\alpha$2/omega1(Gly)-(BglII) der Formel

```
                   5                        10                       15
Cys Asp Leu Pro Gln Thr His Ser Leu Gly Ser Arg Arg Thr Leu
                  20                        25                       30
Met Leu Leu Ala Gln Met Arg Arg Ile Ser Leu Phe Ser Cys Leu
                  35                        40                       45
Lys Asp Arg Arg Asp Phe Gly Phe Pro Gln Glu Glu Phe Gly Asn
                  50                        55                       60
Gln Phe Gln Lys Ala Glu Thr Ile Pro Val Leu His Glu Met Ile
                  65                        70                       75
Gln Gln Ile Phe Ser Leu Phe His Thr Glu Arg Ser Ser Ala Ala
                  80                        85                       90
Trp Asn Met Thr Leu Leu Asp Gln Leu His Thr Gly Leu His Gln
                  95                       100                      105
Gln Leu Gln His Leu Glu Thr Cys Leu Leu Gln Val Val Gly Glu
                 110                       115                      120
Gly Glu Ser Ala Gly Ala Ile Ser Ser Pro Ala Leu Thr Leu Arg
                 125                       130                      135
Arg Tyr Phe Gln Gly Ile Arg Val Tyr Leu Lys Glu Lys Lys Tyr
                 140                       145                      150
Ser Asp Cys Ala Trp Glu Val Val Arg Met Glu Ile Met Lys Ser

                 155                      160                  165
Leu Phe Leu Ser Thr Asn Met Gln Glu Arg Leu Arg Ser Lys Asp
                 170
Arg Asp Leu Gly Ser Ser
```

, dessen Met- und N-Formyl-Met-Derivat sowie dessen N-glykosyliertes Derivat, und enthält die für dieses Peptid kodierende Sequenz der Formel

```
ATG TGT GAT CTG CCT CAA ACC CAC AGC CTG GGT AGC AGG AGG ACC  45
TTG ATG CTC CTG GCA CAG ATG AGG AGA ATC TCT CTT TTC TCC TGC  90
TTG AAG GAC AGA CGT GAC TTT GGA TTT CCC CAG GAG GAG TTT GGC 135
AAC CAG TTC CAA AAG GCT GAA ACC ATC CCT GTC CTC CAT GAG ATG 180
ATC CAG CAG ATC TTC AGC CTC TTC CAC ACA GAG CGC TCC TCT GCT 225
GCC TGG AAC ATG ACC CTC CTA GAC CAA CTC CAC ACT GGA CTT CAT 270
CAG CAA CTG CAA CAC CTG GAG ACC TGC TTG CTG CAG GTA GTG GGA 315
GAA GGA GAA TCT GCT GGG GCA ATT AGC AGC CCT GCA CTG ACC TTG 360
AGG AGG TAC TTC CAG GGA ATC CGT GTC TAC CTG AAA GAG AAG AAA 405
TAC AGC GAC TGT GCC TGG GAA GTT GTC AGA ATG GAA ATC ATG AAA 450
TCC TTG TTC TTA TCA ACA AAC ATG CAA GAA AGA CTG AGA AGT AAA 495
GAT AGA GAC CTG GGC TCA TCT TGA                             519
```

oder deren degenerierte Varianten.

Zur Herstellung eines für ein Hybridinterferon der Formel I kodierenden Plasmids, das vor der gemeinsamen BglII-Schnittstelle die für ein omega1-Interferon kodierende DNA-Sequenz enthält (Konstruktionsschema: siehe Figur 5), muß, falls in der DNA-Sequenz des $\alpha$-Interferons zwei BglII-Schnittstellen wie in der DNA-Sequenz des IFN-$\alpha$2(Arg) vorhanden sind, in zwei Schritten verfahren werden: Im ersten Schritt wird das aus dem Plasmid pRHW14 durch Schnitt mit BglII und SphI gewonnene große Fragment mit dem aus dem Plasmid parpATER33 durch Verdauen mit BglII und SphI gewonnene Fragment, welches den C-Terminus des IFN-$\alpha$2(Arg) kodiert, in Gegenwart einer Ligase, z.B. $T_4$ DNA-Ligase, ligiert. Zur Replikation der entstandenen Plasmide wurden Bakterien, vorzugsweise E. coli HB 101, wie vorstehend bei der Herstellung des Plasmids pRH72 beschrieben, transformiert, kultiviert und auf ihre Konstruktion hin überprüft.

Ein so erhaltenes Plasmid wurde ausgewählt und mit pRH77 bezeichnet, dieses weist folgende Restriktionskarte auf:

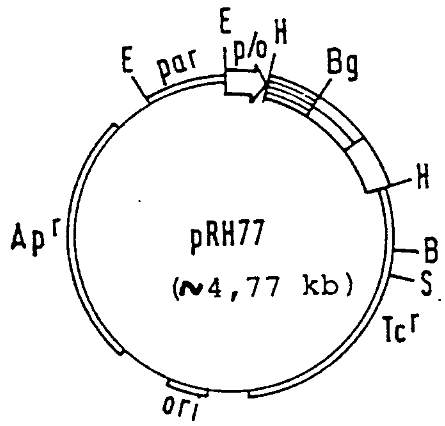


Im 2. Schritt muß nunmehr, um das Gen für das Hybridinterferon, z.B. des IFN-omega1/$\alpha$2(BglII), zu komplettieren, das beim Schneiden von parpATER33 entfernte Fragment mit den Positionen 192-455 wieder in das Plasmid pRH77 eingebracht werden. Hierzu wird das Plasmid pRH77 mit BglII geschnitten, und das 5'-terminale Phosphat mit Kalbsdarm-Phosphatase (CIP) entfernt. Die so erhaltene linearisierte Form des Plasmids pRHW77 wurde durch Gelelektrophorese, z.B. mit einem 1%igen Agarosegel, aufgetrennt, isoliert und mittels Äthanol-Präzipitation gereinigt. Nach Lösen des so erhaltenen linearisierten Fragments in einem geeigneten Puffer, z.B. in TE-Puffer, wurde dieses mit dem beim ursprünglichen Verdauen von parpATER33 mit BglII und SphI erhaltenen 263 bp langen Fragment in Gegenwart einer Ligase, z.B. $T_4$ DNA-Ligase, ligiert. Zur Replikation der entstandenen Plasmide werden Bakterien, vorzugsweise E. coli HB101, wie

vorstehend bei der Herstellung des Plasmids pRH72 beschrieben, transformiert, kultiviert und auf ihre Konstruktion hin durch Schnitt mit den Restriktionsendonucleasen AluI oder HaeIII auf ihre Richtigkeit hin untersucht. Die Insertion des 263 pb langen Fragments erfolgt hierbei auf Grund der identischen Enden in zwei Orientierungen.

Das Plasmid, in dem das 263 bp lange BglII-Fragment in der für die Expression richtigen Lage insertiert wurde, wurde mit pRH78r, und jenes mit der für die Expression falschen Orientierung mit pRH78f bezeichnet.

Beide Plasmide zeigen transformiert in E. coli HB101 den Phänotyp $Ap^r$ und $Tc^r$. Das Plasmid pRH78r mit der Restriktionskarte

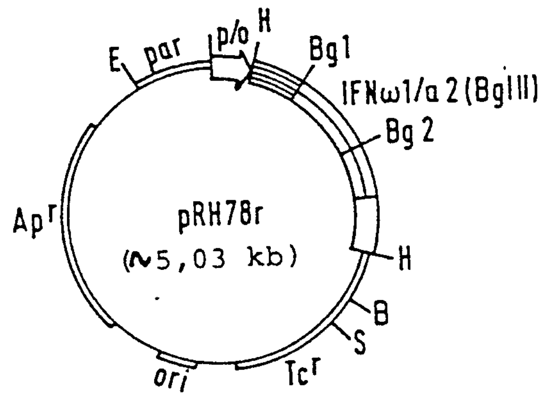


kodiert folgende Polypeptidesequenz für das IFN-omega1/α2(BglII) und enthält die für dieses Peptid kodierende Sequenz:

| | | | | | | | | | | | | | | | |
|---|---|---|---|---|---|---|---|---|---|---|---|---|---|---|---|
| | | | | 5 | | | | | 10 | | | | | 15 | |
| Met | Cys | Asp | Leu | Pro | Gln | Asn | His | Gly | Leu | Leu | Ser | Arg | Asn | Thr | |
| ATG | TGT | GAT | CTG | CCT | CAG | AAC | CAT | GGC | CTA | CTT | AGC | AGG | AAC | ACC | 45 |
| | | | | 20 | | | | | 25 | | | | | 30 | |
| Leu | Val | Leu | Leu | His | Gln | Met | Arg | Arg | Ile | Ser | Pro | Phe | Leu | Cys | |
| TTG | GTG | CTT | CTG | CAC | CAA | ATG | AGG | AGA | ATC | TCC | CCT | TTC | TTG | TGT | 90 |

```
                    35                  40                  45
Leu Lys Asp Arg Arg Asp Phe Arg Phe Pro Gln Glu Met Val Lys
CTC AAG GAC AGA AGA GAC TTC AGG TTC CCC CAG GAG ATG GTA AAA 135
                    50                  55                  60
Gly Ser Gln Leu Gln Lys Ala His Val Met Ser Val Leu His Glu
GGG AGC CAG TTG CAG AAG GCC CAT GTC ATG TCT GTC CTC CAT GAG 180
                    65                  70                  75
Met Leu Gln Gln Ile Phe Asn Leu Phe Ser Thr Lys Asp Ser Ser
ATG CTG CAG CAG ATC TTC AAT CTC TTC AGC ACA AAG GAC TCA TCT 225
                    80                  85                  90
Ala Ala Trp Asp Glu Thr Leu Leu Asp Lys Phe Tyr Thr Glu Leu
GCT GCT TGG GAT GAG ACC CTC CTA GAC AAA TTC TAC ACT GAA CTC 270
                    95                  100                 105
Tyr Gln Gln Leu Asn Asp Leu Glu Ala Cys Val Ile Gln Gly Val
TAC CAG CAG CTG AAT GAC CTG GAA GCC TGT GTG ATA CAG GGG GTG 315
                    110                 115                 120
Gly Val Thr Glu Thr Pro Leu Met Lys Glu Asp Ser Ile Leu Ala
GGG GTG ACA GAG ACT CCC CTG ATG AAG GAG GAC TCC ATT CTG GCT 360
                    125                 130                 135
Val Arg Lys Tyr Phe Gln Arg Ile Thr Leu Tyr Leu Lys Glu Lys
GTG AGG AAA TAC TTC CAA AGA ATC ACT CTC TAT CTG AAA GAG AAG 405
                    140                 145                 150
Lys Tyr Ser Pro Cys Ala Trp Glu Val Val Arg Ala Glu Ile Met
AAA TAC AGC CCT TGT GCC TGG GAG GTT GTC AGA GCA GAA ATC ATG 450
                    155                 160                 165
Arg Ser Phe Ser Leu Ser Thr Asn Leu Gln Glu Ser Leu Arg Ser
AGA TCT TTT TCT TTG TCA ACA AAC TTG CAA GAA AGT TTA AGA AGT 495

Lys Glu
AAG GAA TGA                                                 504
```

Das Plasmid pRH78f mit der Restriktionskarte

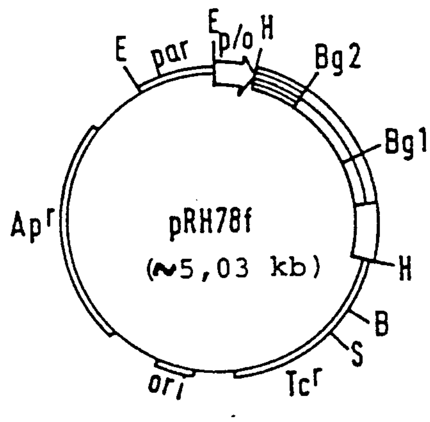


kodiert folgende Polypeptidesequenz und enthält die für dieses Peptid kodierende Sequenz:

```
                    5                   10                  15
Met Cys Asp Leu Pro Gln Asn His Gly Leu Leu Ser Arg Asn Thr
ATG TGT GAT CTG CCT CAG AAC CAT GGC CTA CTT AGC AGG AAC ACC  45
                    20                  25                  30
Leu Val Leu Leu His Gln Met Arg Arg Ile Ser Pro Phe Leu Cys
TTG GTG CTT CTG CAC CAA ATG AGG AGA ATC TCC CCT TTC TTG TGT  90
                    35                  40                  45
Leu Lys Asp Arg Arg Asp Phe Arg Phe Pro Gln Glu Met Val Lys
CTC AAG GAC AGA AGA GAC TTC AGG TTC CCC CAG GAG ATG GTA AAA 135
                    50                  55                  60
Gly Ser Gln Leu Gln Lys Ala His Val Met Ser Val Leu His Glu
GGG AGC CAG TTG CAG AAG GCC CAT GTC ATG TCT GTC CTC CAT GAG 180
                65
Met Leu Gln Gln Ile Ser
ATG CTG CAG CAG ATC TCA TGA TTT CTG CTC TGA CAA CCT CCC AGG 225
CAC AAG GGC TGT ATT TCT TCT CTT TCA GAT AGA GAG TGA TTC TTT 270
GGA AGT ATT TCC TCA CAG CCA GAA TGG AGT CCT CCT TCA TCA GGG 315
GAG TCT CTG TCA CCC CCA CCC CCT GTA TCA CAC AGG CTT CCA GGT 360
CAT TCA GCT GCT GGT AGA GTT CAG TGT AGA ATT TGT CTA GGA GGG 405
TCT CAT CCC AAG CAG CAG ATG AGT CCT TTG TGC TGA AGA GAT TGA 450
AGA TCT TTT TCT TTG TCA ACA AAC TTG CAA GAA AGT TTA AGA AGT 505
AAG GAA TGA                                                 514
```

Zur Replikation bzw. zur Expression können die neuen Plasmide der vorliegenden Erfindung in einen bakteriellen Wirt eingebracht werden. Hierbei haben sich Prokaryoten wie E. coli K12, Stamm 294 (ATCC Nr. 31.446), E. coli X1776 (ATCC Nr. 31.537), E. coli W3110 ($F^-$, Lambda$^-$, Prototroph, ATCC Nr. 27.325), E. coli HB101 (($F^-$, hsdS20($r^-$, $m^-$), recA13, ara-14, proA2, lacY1, galK2, rpsL20(Smr), xyl-5, mtl-1, supE44, lambda$^-$), Bazillen wie Bacillus subtilis, und andere Enterobacteriaceae, wie Salmonella typhimurium oder Serratia marcenses und verschiedene Pseudomonaden als geeignet erwiesen.

Zur Expression der neuen Hybridinterferone wurde beispielsweise das Plasmid pRH72, pRH78f und pRH78r jeweils in E. coli transformiert und kultiviert. Die antivirale Aktivität der exprimierten Polypeptide

wurde im Zellüberstand nach der Zerstörung der Zellwände und Abzentrifugation der Bakterientrümmer und mittels des CPE-Reduktionstest bestimmt. Hierbei zeigte sich, daß die aus E. coli transformiert mit pRH72 und E. coli transformiert mit pRH78f gewonnenen Zellüberstände keine antiviralen Eigenschaften aufweisen. Überraschenderweise weist jedoch der aus dem Plasmid pRH78r gewonnene Zellüberstand, der das IFN-omega1/α2(BglII) enthält, eine etwa viermal höhere spezifische antivirale Aktivität auf A549-Zellen als IFN-α2(Arg) auf.

Die für ein neuen Hybridinterferon kodierenden DNA-Sequenzen der neuen erfindungsgemäß hergestellten Plasmide können desweiteren nach entsprechender Veränderung in jeden anderen Organismus eingebracht werden.

Hierbei kann die Expression und Translation einer derartigen Sequenz auch unter Kontrolle anderer Regulationssysteme, die als "homolog" zu dem Organismus in seiner untransformierten Form gelten können, ablaufen. So enthält z.B. chromosomale DNA von einem Lactose-abhängigen E. coli ein Lactose oder Lac-Operon, das durch Ausschüttung des Enzyms Beta-Galactosidase den Lactose-Abbau ermöglicht.

Die Lac-Kontrollelemente können aus dem Bacteriophagen Lambda-plac5, der infektös für E. coli ist, erhalten werden. Das Lac-Operon des Phagen kann durch Transduktion aus derselben Bakterien-Spezies stammen. Regulationssysteme, die bei dem erfindungsgemäßen Verfahren Verwendung finden können, können auch aus plasmidischer DNA stammen, die dem Organismus eigen ist. Das Lac-Promotor-Operator-System kann durch IPTG (Isopropylthiogalactosid) induziert werden.

Andere Promotor-Operator-Systeme oder Teile hiervon können genausogut verwendet werden: beispielsweise Arabinose-Promotor/Operator, Colicin $E_1$-Promotor/Operator, Galactose-Promotor/Operator, alkalischer Phosphatase-Promotor/Operator, trp-Promotor/Operator, Xylose-A-Promotor/Operator, tacPromotor u.ä..

Zusätzlich zu Prokaryoten können auch Eukaryoten, wie Hefe verwendet werden. Saccharomyces cerevisiae ist die am meisten verwendete unter den eukaryotischen Mikroorganismen, obwohl eine Anzahl anderer Spezies allgemein erhältlich ist. Zur Expression in Saccharomyces wird beispielsweise das Plasmid YRp7 (Stinchcomb et al. Natur 282, 39 (1979); Kingsman et al., Gene 7, 141 (1979); Tschumper et al., Gene 10, 157 (1980)) und das Plasmid YEp 13 (Bwach et al., Gene 8, 121-133 (1979)) üblicherweise verwendet. Das Plasmid YRp7 enthält das TRP1-Gen, das einen Selektionsmarker für eine Hefemutante bereitstellt, die unfähig ist, in trypthophanlosem Medium zu wachsen; beispielsweise ATCC Nr. 44076.

Das Vorhandensein des TRP1-Schadens als Charakteristikum des Hefe-Wirtsgenoms stellt dann ein wirksames Hilfsmittel dar, um die Transformation nachzuweisen, indem ohne Tryptophan kultiviert wird. Ganz ähnlich verhält es sich bei dem Plasmid YEp13, das das Hefe-Gen LEU 2, das zur Ergänzung einer LEU-2-minus-Mutante verwendet werden kann, enthält. Geeignete Promotor-Sequenzen für Hefe Vektoren beinhalten die 5'-flankierende Region der Gene des ADH I (Ammerer G., Methods of Enzymology 101, 192-201 (1983)), 3-Phosphoglycerate-Kinase (Hitzeman et al., J. Biol. Chem. 255, 2073 (1980)) oder andere glykolytische Enzyme (Kawaski und Fraenkel, BBRC 108, 1107-1112 (1982)) wie Enolase, Glyceraldehyd-3-phosphat-Dehydrogenase, Hexokinase, Pyruvat-Decarboxylase, Phosphofructokinase, Glucose-6-Phosphat-Isomerase, Phosphoglucose-Isomerase und -Glucokinase. Bei der Konstruktion geeigneter Expressionsplasmide können die mit diesen Genen assoziierten Terminationssequenzen ebenfalls in den Expressions-Vektor am 3'-Ende der zu exprimierenden Sequenz eingesetzt werden, um Polyadenylierung und Termination der mRNA vorzusehen.

Andere Promotoren, die zudem noch den Vorteil der durch Wachstumsbedingungen kontrollierten Transkription besitzen, sind die Promotor-Regionen der Gene für Alkohol-Dehydrogenase-2, Isocytochrom C, Saure-Phosphatase, abbauende Enzyme, die mit dem Stickstoff-Metabolismus gekoppelt sind, die oben erwähnte Glyceraldehyd-3-Phosphat-Dehydrogenase und Enzyme, die für die Verarbeitung von Maltose und Galaktose verantwortlich sind. Promotoren, die durch den Hefe Mating Typ Locus reguliert werden, beispielsweise Promotoren der Gene BARI, MFα1, STE2, STE3, STE5 können bei temperaturregulierten Systemen durch die Verwendung von temperaturabhängigen sir Mutationen eingesetzt werden. (Rhine PH.D. in Thesis, University of Oregon, Eugene, Oregon (1979), Herskowitz and Oshima, The Molecular Biology of the Yeast Saccharomyces, part I, 181-209 (1981), Cold Spring Harbor Laboratory). Diese Mutationen beeinflussen die Expression der ruhenden Mating Typ Kassetten von Hefen und dadurch indirekt die Mating Typ abhängigen Promotoren. Generell ist jedoch jeder Vektor geeignet, der einen Hefe-kompatiblen Promotor, sowie Hefe spezifische Replikations- und Terminationssequenzen enthält.

Zusätzlich zu Mikroorganismen sind Kulturen multizellulärer Organismen ebenfalls geeignete Wirtsorganismen. Im Prinzip ist jede dieser Kulturen einsetzbar, ob von Wirbeltier- oder wirbellosen Zellkulturen. Größtes Interesse besteht jedoch an Wirbeltier-Zellen, so daß die Vermehrung von Wirbeltierzellen in Kultur (Gewebe-Kultur) in den letzten Jahren zu einer routinemäßigen Methode wurde (Tissue Culture, Academic Press, Kruse and Patterson, Editors (1973)). Beispiele solcher nützlichen Wirtszellinien sind VERO- und

HeLa-Zellen, Goldhamster-Eierstock (CHO)-Zellen und W138, BHK, COS-7 und MDCK-Zellinien. Expressionsvektoren für diese Zellen enthalten üblicherweise einen Replikationsursprung, einen Promotor, der vor dem zu exprimierenden Gen lokalisiert ist, gemeinsam mit der notwendigen RNA-Splicing-Stelle, Polyadenylierungsstelle und transkriptionelle Terminations-Sequenzen.

Bei der Verwendung in Säugetierzellen werden die Kontrollfunktionen auf den Expressions-Vektoren oftmals aus viralem Material verwendet. Beispielsweise stammen die üblicherweise verwendeten Promotoren aus Polyoma Adenovirus 2, und besonders häufig aus Simian Virus 40 (SV 40). Die Promotoren der frühen und späten Gene des SV 40 sind besonders nützlich, da beide leicht aus dem Virus als Fragment zu erhalten sind, das auch noch den viralen Replikationsursprung des SV 40 enthält. (Fiers et al., Nature 273, 113 (1978)). Auch können kleinere oder größere Fragmente des SV 40 verwendet werden, vorausgesetzt, sie enthalten die annähernd 250 bp lange Sequenz, die von der HindIII Schnittstelle bis zur Bgl 1 Schnittstelle in der viralen Replikationsstelle reicht. Außerdem ist es ebenfalls möglich und oft empfehlenswert, Promotor- oder Kontroll-Sequenzen zu verwenden, die normalerweise mit den gewünschten Gensequenzen verknüpft sind, vorausgesetzt, diese Kontroll-Sequenzen sind kompatibel zu den Wirtszellsystemen.

Eine Replikationsstelle kann entweder durch entsprechende Vektorkonstruktion vorgesehen werden, um eine exogene Stelle einzubauen, beispielsweise aus SV 40 oder anderen viralen Quellen (z.B. Polyoma, Adeno, VSV, PBV, etc.) oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Basierend auf ihrem biologischen Wirkungsspektrum sind die neuen, erfindungsgemäßen Hybride, insbesondere das IFN-omega1/$\alpha$2(BglII) Interferon verwendbar für jede Art der Behandlung, für die auch die bekannten Interferone eingesetzt werden. Diese beinhalten beispielsweise Herpesvirus, Rhinovirus, virale Infektionen bei AIDS sowie andere virale Erkrankungen, verschiedene Krebsarten und Ähnliches. Die neuen Interferone können alleine oder in Kombination mit anderen bekannten Interferonen oder biologisch aktiven Produkten eingesetzt werden, beispielsweise mit IFN-$\gamma$, IL-2, anderen Immun-Modulatoren und Ähnlichen.

Ein IFN-omega/$\alpha$-Hybrid wie IFN-omega1/$\alpha$2(BglII) kann parenteral verabreicht werden in Fällen, in denen Antitumor oder antivirale Behandlung erforderlich ist, und zur antiviralen Prophylaxe in immunsupprimierten Patienten. Dosierung und Dosierungsrate können ähnlich denen sein, die zur Zeit bei klinischen Untersuchungen für IFN-$\alpha$-Materialien gelten z.B. ca. (1-10) x $10^6$ Einheiten täglich und bei Präparaten, die zu mehr als 1 % rein sind, bis zu beispielsweise 5 x $10^7$-Einheiten täglich.

Beispielsweise können für eine zweckmäßige Dosierungsform bei einem im wesentlichen homogenen, bakteriell produzierten IFN-omega1/$\alpha$2(BglII), bei parenteraler Verwendung 3 mg IFN-omega1/$\alpha$2(BglII) in 25 ml 5%igem menschlichem Serum Albumin gelöst werden. Diese Lösung wird dann durch ein bakteriologisches Filter gegeben, und die gefilterte Lösung aseptisch auf 100 Fläschchen verteilt, von dem jedes 6 x $10^6$ Einheiten reines IFN-omega1/$\alpha$2(BglII) zur parenteralen Applikation geeignet, enthält. Die Gläschen werden vor Verwendung vorzugsweise kühl (-20 °C) aufbewahrt. Die erfindungsgemäßen Substanzen können in bekannter Weise formuliert werden, um pharmazeutisch verwendbare Mittel zu erhalten, wobei das erfindungsgemäße Polypeptid mit einer pharmazeutischen, akzeptablen Trägersubstanz vermischt wird. Gebräuchliche Trägerstoffe und ihre Formulierung sind bei E.W. Martin in Remingtom's Pharmaceutical Sciences beschrieben, worauf ausdrücklich Bezug genommen wird. IFN-omega1/$\alpha$2(BglII) wird mit einer angemessenen Menge des Tragerstoffes vermischt, um geeignete pharmazeutische Mittel zu schaffen, die für eine effektive Anwendung beim Empfänger (Patienten) geeignet sind. Bevorzugt wird eine parenterale Applikation vorgenommen.

Desweiteren eignen sich die neuen Hybridinterferone zur Herstellung von Antikörpern gegen diese Interferone, insbesondere das IFN-omega1/$\alpha$2(BglII), die zugleich auch die parenteralen Interferone IFN-$\alpha$2 und IFN-omega1 erkennen. Die so hergestellten Antikörper sind daher für die Immunaffinitätschromatographie von omega-Interferonen geeignet.

Die für die Durchführung der vorliegenden Anmeldung erforderlichen Basisplasmide sind in Sachen der EP-A-0.115.613 bzw. der EP-A-0.170.204 bei der Deutschen Sammlung für Mikroorganismen hinterlegt worden, z.B.

pER103 unter der DMS-Nr. 2773 am 20. Dezember 1983,

E76E9 unter der DMS-Nr. 3003 und

P9A2 unter der DMS-Nr. 3004 jeweils am 4. Juli 1984,

wobei für diese Klone bereits die erforderliche Freigabeerklärung abgegeben wurde. Unter Verwendung dieser Plasmide ist es dem Fachmann auf diesem Gebiet ohne weiteres möglich den Gegenstand der vorliegenden Erfindung an Hand der EP-A-0.115.613 und der EP-A-0.170.204 (siehe auch Nucleic Acids Res. 13, 4739-4749 (1985)) nachzuarbeiten.

Die nachfolgenden Beispiele sollen die Erfindung näher erläutern, ohne diese jedoch einzuschränken:

Vorbemerkung

Alle Enzymreaktionen werden unter den vom Hersteller angegebenen Bedingungen durchgeführt.

Beispiel 1:

Herstellung von parpATER33

10 µg parpER33 werden in 200 µl Reaktionslösung mit je 20 Einheiten BamHI und PstI geschnitten. Anschließend werden die zwei entstehenden Fragmente in einem 1%igen Agarosegel aufgetrennt (1% Agarose in 1x TBE-puffer: 10,8 g/l Tris(hydroxymethyl)aminomethan (Tris), 5,5 g/l Borsäure, 0,93 g/l Äthylendinitrilo-tetraessigsäure-dinatriumsalz (EDTA) und 0,5 mg/l Äthidiumbromid (EtBr), Laufpuffer ist 1x TBE; Elektrophorese bei ca. 5V/cm; Sichtbarmachen der DNA-Fragmente durch Bestrahlung des Agarosegels mit UV-Licht (254 nm)). Das kleinere, das Interferon-alpha2-Arg (IFN-$\alpha$2(Arg)) enthaltende Fragment wird isoliert (Elektrophorese der DNA-Bande auf DE-81 Papier (Whatman), Waschen des Papiers mit 200 mM NaCl, 25 mM Tris pH = 7,5, 1 mM EDTA und Elution der DNA mit 1 M NaCl, 25 mM Tris, pH = 7,5, 1 mM EDTA) und durch Zusatz von 2,5 Vol Äthanol wird die DNA ausgefällt. Nach dem Abzentrifugieren wird die DNA getrocknet und in einem geeigneten Volumen TE-Puffer (10 mM Tris pH = 8,0, 1 mM EDTA) aufgenommen.

10 µg pAT153 werden ebenfalls in 200 µl Reaktionslösung mit je 20 Einheiten BamHI und PstI geschnitten, und die zwei entstehende Fragmente separiert. Von pAT153 isoliert man das größere, den Replikationsursprung enthaltende Fragment.

Jeweils 0,5 µg der gereinigten DNA Fragmente werden in 20 µl Reaktionslösung (66 mM Tris, pH = 7,5, 100 mM NaCl, 10 mM $MgCl_2$, 5 mM Dithiothreit (DTT), 1 mM EDTA, 1 mM Adenosintriphosphat (ATP)) mit 5 Einheiten $T_4$ DNA-Ligase ligiert. Anschließend werden 150 µ1 kompetente E.coli HB 101-Bakterien ($F^-$, hsdS20($r^-$, $m^-$), recA13, ara-14, proA2, lacY1, galK2, rpsL20(Smr), xyl-5, mtl-1, supE44, $lambda^-$) mit 1 µl der Ligasereaktion versetzt, 30 Minuten bei 0° C inkubiert, und durch 2 Minuten 42° C Inkubation mit der DNA transformiert (Kompetente E.coli Bakterien: E.coli wird in LB-Medium (10 g/l Trypton, 5 g/l Yeast Extract, 5 g/l NaCl, pH = 7,5) bis zu $OD_{600nm}$ = 0,3 wachsen gelassen und abzentrifugiert. Die Bakterien werden in 0,5 Vol eiskalter 50 mM $CaCl_2$ Lösung resuspendiert und 30 Minuten inkubiert. Nach erneutem Abzentrifugieren werden die Bakterien in 1/15 Vol des Ausgangsvolumens 50 mM $CaCl_2$ resuspendiert). Die Bakteriensuspension wird auf LB-Agar (LB-Medium plus 15 g/l Agar) mit 50 µg/ml Ampicillin plattiert. Nach 16 Stunden Inkubation bei 37° C werden 12 der entstandenen Kolonien ausgewählt und von diesen im Mikromaßstab die Plasmide isoliert (Birnboim, H.C. and Doly, J., Nucl. Acids Res. 7, 1513 (1979)). Die Richtigkeit der Konstruktion wird durch Restriktionsenzymdoppelverdauung mit PstI-BamHI, PstI-PvuII und EcoRI-BamHI und mit anschließender Gelelektrophorese durch das Auftreten der erwarteten Fragmente bestätigt. Ein Plasmid wird ausgewählt und mit parpATER33 bezeichnet. E.coli transformiert mit parpATER33 zeigt den Phänotyp $Ap^r$ (Ampicillin-Resistenz), $Tc^r$ (Tetracyclin-Resistenz).

Beispiel 2

Herstellung von pRHW14

10 µg parpATER33 werden in 150 µl Reaktionslösung mit HindIII und BamHI doppelt verdaut. Die drei resultierenden DNA Fragmente werden auf einem 1%igen Agarosegel aufgetrennt, und das größte, ca. 3750 Basenpaare (bp) lange Fragment isoliert (Fragment a)). Dieses Fragment trägt den Tryptophan Promotor/Operator (Serratia marcescens), den Replikationsursprung, sowie das $Ap^r$-Gen.

10 µg pRHW12 werden ebenfalls in 150 µl Reaktionslösung mit BamHI und HindIII doppelt verdaut. Die zwei entstehenden Fragmente werden gelelektrophoretisch aufgetrennt, und das kleinere, ca. 800 bp lange Fragment isoliert, welches das IFN-omega1(Gly)-Gen enthält (Fragment b)).

40 ng Fragment a) werden mit ca. 50 ng Fragment b) in 10 µl Reaktionslösung mit 5 Einheiten $T_4$ DNA-Ligase ligiert. 200 µl kompetenter E.coli HB101 Suspension werden mit der Ligasereaktionslösung gemischt, die Bakterien durch Hitzeschock auf 42° C transformiert und auf LB-Agar plus 50 µg/ml Ampicillin plattiert. Nach 16 Stunden Inkubation bei 37° C werden sechs Kolonien ausgewählt, und aus den Bakterien im Mikromaßstab die Plasmid DNA isoliert. Nach dem Verdauen der DNA mit den Restriktionsendonukleasen EcoRI, HindIII, NcoI bzw. PstI und anschließender gelelektrophoretischer Analyse der Fragmente zeigte

sich, daß eines der Plasmide die gewünschte Struktur aufweist. Dieses Plasmid wird mit pRHW14 bezeichnet. E.coli transformiert mit pRHW14 zeigt den Phänotyp $Ap^r$ und $Tc^s$.

Beispiel 3

Nachweis der Plasmid-kodierten Proteine

Der Nachweis Plasmid-kodierter Proteine ist im Maxizell System möglich (Sancar. A., Hack, A.M. and Rupp, W.D., J.Bacteriol. 137, 692-693 (1979)).
Hierzu wird E.coli CSR603 ($F^-$, thr-1, leuB6, proA2, phr-1, recA1, argE3, thi-1, uvrA6, ara-14, lacY1, galK2, xyl-5, mtl-1, gyrA98 (nalA98), rpsL31, tsx-33, $lambda^-$, supE44), transformiert mit den Plasmiden pRHW12 und pRHW14, im Expressionsmedium (10 g/l $(NH_4)_2PO_4$, 3,5 g/l $KH_2PO_4$ pH = 7,3, 0,5 g/l NaCl, 21 g/l Caseinhydrolysat (säurehydrolysiert, vitaminfrei), 11 g/l Glucose, 1 mM $MgSO_4$, 0,1 mM $CaCl_2$, 1 mg/l Thiamin-HCl, 20 mg/l L-Cystein, 100 mg/l Ampicillin) bis zu einer Dichte von $OD_{600}$nm = 0,6 bei 37° C gezüchtet. 10 ml Kultur werden in einer offenen Petrischale mit einer UV-Germicidlampe (15W) aus 50 cm Entfernung 5 Sekunden lang bestrahlt und eine Stunde weiter inkubiert. Den Kulturen werden 100 µg D-Cycloserin zugesetzt, um noch vermehrungsfähige Bakterien abzutöten. Nach 16 Stunden Inkubation bei 37° C werden die Bakterien abzentrifugiert, zweimal mit je 5 ml Hershey-Salzlösung gewaschen (5,4 g/l NaCl, 3,0 g/l KCl, 1,1 g/l $NH_4Cl$, 15 mg/l $CaCl_2x2H_2O$, 0,2 g/l $MgCl_2x6H_2O$, 0,2 mg/l $FeCl_3x6H_2O$, 87 mg/l $KH_2PO_4$, 12,1 g/l Tris pH = 7,4), in 5 ml Hershey-Medium (pro 100 ml Hershey-Salze: 2,0 ml 20% Glucose, 0,5 ml 2% Threonin, 1,0 ml 1 % Leucin, 1,0 ml 2% Prolin, 2% Arginin, 0,1 ml 0,1% Thiamin-HCl) plus 20 µg/ml Indolacrylsäure (IAA) inkubiert. Durch Zusatz von 5 µCi/ml $^{35}S$-Methionin und weiterer Inkubation bei 37° C für eine Stunde werden neusynthetisierte Proteine radioaktiv markiert. Die Bakterien werden abzentrifugiert und in 200 µl Na-Dodecylsulfat(SDS)-Probenpuffer (6,6 mM Na-Phosphat pH = 6,8, 2 mM EDTA, 2% SDS, 3% Glyzerin, 0,02% Bromphenolblau, 0,66% 2-Mercaptoäthanol) 5 Minuten bei 100° C lysiert. Die Proben werden anschließend in einem 15%igem Polyacrylamidgel aufgetrennt (Trenngel: 15% Acrylamid, 0,4% Bisacrylamid, 375 mM Tris pH = 8,8, 2 mM EDTA, 0,1% SDS; Sammelgel: 6% Acrylamid, 0,16% Bisacrylamid, 375 mM Tris pH = 6,8, 2 mM EDTA, 0,1% SDS; Elektrodenpuffer: 3,0 g/l Tris, 14,24 g/l Glyzin, 0,335 g/l EDTA, 0,5 g/l SDS; Elektrophoresedauer: 16 Stunden bei konstant 20 mA). Das Gel wird eine Stunde in 20% Methanol, 7,5% Essigsäure fixiert, 30 Minuten in 5% Methanol, 1% Glyzerin inkubiert und in einem Geltrockner getrocknet. Das Gel wird unter Verwendung einer Verstärkerfolie (Kodak) bei -80° C auf Kodak X-Omat S Röntgenfilm exponiert.

Das Ampicillin-Resistenzgenprodukt (β-Lactamase) wird in beiden Fällen gleich stark markiert. IFN-omega1 ist jedoch im Falle des pRHW14 mehr als doppelt so stark markiert als bei pRHW12 (siehe Figur 3).

Beispiel 4

Extraktion von IFN-omega1(Gly) aus Bakterien

E.coli HB101 transformiert mit pRHW12 bzw. pRHW14 wird in Expressionsmedium plus 20 µg/ml IAA bis zu einer $OD_{600}$nm = 20 angezüchtet. Die Bakterien werden durch Zusatz von $H_2SO_4$ bis pH = 2 und 60-minütige Inkubation bei 20° C abgetötet. Die Bakterien werden abzentrifugiert, und die Biomasse bei -20° C bis zur Aufarbeitung eingefroren. Die Bakterien werden in 10 Vol 1% Essigsäure resuspendiert. Der pH-Wert der Lösung wird durch Zusatz von 2 N NaOH auf 10 eingestellt, und die Suspension bei 0° C zwei Stunden gerührt. Danach wird mit 2 N HCl wieder ein pH-Wert von 7,5 eingestellt und die Zelltrümmer abzentrifugiert (J2-21 Zentrifuge (Beckman), JA10 Rotor, 4° C, 10000 rpm, 30 Minuten). Die Interferon-Aktivität im Überstand (Rohextrakt) wird mittels des Cytopathischen Effekt (CPE) Reduktionstests auf humanen A549 Zellen (menschliche Lungenkarzinom Zellinie), mit Encephalomyocarditis (EMC) Virus infiziert, unter Verwendung von IFN-α2(Arg) als Standard gemessen. Dabei liefert die Biomasse von E.coli transformiert mit pRHW12 100.000 Einheiten/Gramm (Mittelwert aus drei unabhängigen Züchtungen), während der Klon pRHW14 200.000 Einheiten/Gramm Biomasse (15 Züchtungen) liefert.

Beispiel 5

Konstruktion des Hybridinterferon Expressionsklons pRH72

10 µg parpATER33 bzw. pRHW14 werden in je 130 µl Lösung mit BglII und SphI doppelt verdaut. Die

entstandenen Fragmente werden auf einem 0,8% Agarosegel aufgetrennt, die DNA's eluiert und durch Präzipitation gereinigt. Die Fragmente werden in 20 µl TE gelöst. Das Expressionsplasmid für IFN-α2/omega1(BglII) wird durch Ligasereaktion von 1 µl großem Fragment aus parpATER33 mit 5 µl kleinem Fragment von pRHW14 in insgesamt 20 µl unter Verwendung von 5 Einheiten $T_4$ DNA-Ligase hergestellt. Nach der Transformation von E.coli HB101 werden die Plasmide einiger der resultierenden, Ampicillin resistenten Klone im Mikromaßstab isoliert, und die Richtigkeit der Konstruktion durch doppelte Restriktionsenzymverdauung mit BglII/SphI überprüft. Eines der Plasmide wird ausgewählt und mit pRH72 bezeichnet. E.coli transformiert mit diesem Plasmid besitzt den Phänotyp $Ap^r$, $Tc^s$.

## Beispiel 6

### Konstruktion der Plasmide pRH78r und pRH78f

1 µl des großen Fragments von pRHW14 wird mit 5 µl des BglII(2)-SphI-Fragments aus parpATER33, das den C-Terminus des IFN-α2(Arg) kodiert, in 20 µl Reaktionslösung mit 5 Einheiten $T_4$ DNA-Ligase ligiert. Mit dem entstehenden Plasmid wird E.coli HB101 transformiert und wie in Beispiel 5 beschrieben, ein Plasmid der gewünschten Konstruktion ausgesucht. Dieses Zwischenplasmid wird mit pRH77 bezeichnet. Um das Gen für das Hybridinterferon-omega1/α2(BglII) zu komplettieren, muß das beim Schnitt von parpATER33 mit BglII entfernte Fragment in pRH77 eingebracht werden. Hierzu werden 10 µg pRH77 in 50 µl Lösung mit BglII geschnitten, das Volumen der Lösung mit 2x CIP-Puffer verdoppelt (CIP: Kalbsdarm-Phosphatase, 2x CIP-Puffer: 100 mM Tris pH = 9,0, 2 mM, $MgCl_2$, 0,2 mM $ZnCl_2$), und das 5'-terminale Phosphat durch Zusatz von 1 Einheit CIP (Boehringer Mannheim) entfernt (60 Minuten 37 °C). Die linearisierte Form von pRH77 wird durch Agarosegelelektrophorese und Elution der DNA mit anschließender Präzipitation gereinigt. Die DNA wird in 20 µl TE-Puffer aufgenommen und 1 µl davon mit 5 µl des 263 bp Fragments (BglII(1)-BglII(2)-Fragment), das beim Verdauen des parpATER33 mit BglII/SphI gewonnen wird, in insgesamt 10 µl Reaktionslösung mit 5 Einheiten $T_4$ DNA-Ligase ligiert. E.coli HB101 wird transformiert, und die DNA von sechs entstandenen Kolonien analysiert. Da die Insertion des 263 bp Fragments auf Grund der identischen Enden in zwei Orientierungen erfolgen kann, werden die Plasmide mit den Restriktionsendonukleasen AluI und HaeIII auf die Richtigkeit der Konstruktion hin untersucht. Das Plasmid, in dem das BglII-Fragment in der für die Expression richtigen Lage insertiert wurde, wird mit pRHW78r, jenes mit der für Expression falschen Orientierung mit pRH78f bezeichnet. E.coli transformiert mit pRH78r bzw. pRH78f zeigt den Phänotyp $Ap^r$, $Tc^r$.

## Beispiel 7

### Lysattest zur Feststellung von Interferon-(antiviraler) Aktivität

E.coli transformiert mit den diversen Plasmiden wird in 35 ml Expressionsmedium plus 20 µg/µl IAA bei 37 °C bis zu einer $OD_{600nm}$ = 0,6 angezüchtet. Die Bakterien werden abzentrifugiert. Das Bakterienpellet wird in 3,5 ml 50 mM Tris pH = 7,6/30 mM $MgCl_2$-Lösung aufgenommen und unter Eiskühlung mit einem Ultraschall Desintegrator (MSE, Soniprep 150) mit maximaler Leistung beschallt. Die Suspension wird 10 Minuten zentrifugiert (J2-21 Zentrifuge, 10.000 rpm, 4 °C, JA20-Rotor), und der Überstand steril filtriert. Die antivirale Aktivität der Lösung wird mittels des CPE-Reduktionstests (A549-Zellen, EMC-Virus) bestimmt.

E.coli transformiert mit pRH72 (IFN-α2/omega1(BglII)) ergibt keine antivirale Aktivität ebenso wie E.coli transformiert mit pRH78f, das die ersten 64 Aminosäuren von reifen IFN-omega1, gefolgt von einem Serin kodiert.

Demgegenüber zeigt das IFN-omega1/α2(BglII), wobei der Klon pRH78r ca. $30x10^6$ Einheiten Interferon (verglichen mit IFN-α2(Arg) als Standard) pro Liter Kultur und pro 1 $OD_{600}$nm Bakteriendichte produziert, eine etwa viermal höhere spezifische antivirale Aktivität auf A549 Zellen als IFN-α2(Arg).

In der folgenden Tabelle sind die Aminosäureänderungen des Hybridinterferons IFN-omega1/α2(BglII) gegenüber IFN-α2(Arg) aufgelistet:

| Position | IFN-α2(Arg) | IFN-omega1/α2 | Übergang |
|---|---|---|---|
| 7 | Thr | Asn | p >> p |
| 9 | Ser | Gly | p >> p |
| 11 | Gly | Leu | p >> l |
| 14 | Arg | Asn | b >> p |
| 17 | Met | Val | (l) >> l |
| 20 | Ala | His | l >> b |
| 27 | Leu | Pro | l >> l |
| 29 | Ser | Leu | p >> l |
| 38 | Gly | Arg | p >> b |
| 43 | Glu | Met | a >> (l) |
| 44 | Phe | Val | ar >> l |
| 45 | 0 | Lys | 0 >> b |
| 47 | Asn | Ser | p >> p |
| 49 | Phe | Leu | ar >> l |
| 53 | Glu | His | a >> b |
| 54 | Thr | Val | p >> l |
| 55 | Ile | Met | l >> (l) |
| 56 | Pro | Ser | l >> p |
| 62 | Ile | Leu | l >> l |
| Legende:<br>a: sauer, ar: aromatisch, b: basisch, l: apolar, p: polar 0: keine Aminosäure an dieser Position | | | |

Unterschiede bei den Ladungen (Position):

1) Verlust von 1 positiven Ladung: 14
2) Gewinn von 4 positiven Ladungen: 20, 38, 45, 53
3) Verlust von 2 negativen Ladungen: 43, 53

Das Hybrid hat 5 positive Ladungen mehr als IFN-α2(Arg).

Beispiel 9:

Reinigung des IFN-omega1/α2(BglII)

145 g säurebehandelte und bei -20°C gefrorene E.coli des Klones HB 101/pRH78r werden in 1450 ml 1% Essigsäure bis zur völligen Verteilung des Materials unter Eiskühlung gerührt (etwa 30 Minuten) und sodann 2 x 1 Minute lang mit dem Ultra-Turrax T 45/6 (Janke und Kunkel) bei 10.000 UpM homogenisiert. Anschließend wird Polymin P (Serva, Kat.Nr. 33141) bis zu einer Konzentration von 0,25% zugegeben und der pH mit 5 N NaOH auf 10,0 eingestellt. Nach 2 Stunden Rühren unter Eiskühlung wird der pH mit 5 N HCl auf 7,5 eingestellt und eine Klärung des Rohextraktes durch Zentrifugation vorgenommen (Christ Cryofuge 6-6 S, 3000 UpM, 1 Stunde, etwa 4°C).
Zur Fällung des Interferons wird der Rohextrakt mit 430 g/Liter Ammoniumsulfat versetzt und zur vollständigen Fällung 16 Stunden bei 4-8°C stehen gelassen. Der Niederschlag wird dann durch Zentrifugation gewonnen (Beckman Zentrifuge J 2-21, Rotor JA10, 10.000 UpM, 60 Minuten, 4-8°C). Das Ammonsulfat-Pellet wird in 145 ml 0,01 M NaCl aufgenommen und die Suspension mit 5 N NaOH auf pH 7,5 eingestellt. Nach 3 Stunden Rühren (Eiskühlung) wird die Lösung klarzentrifugiert (Beckman J2-21, Rotor JA10, 10 000 UpM, 4°C, 60 Minuten) und mit Hilfe einer Nephross-Allegro Dialysierpatrone (Fa. Organon) gegen 0,01 M NaCl solange dialysiert, bis die Interferonlösung eine Osmolarität von 370 mOsmol/l aufwies. Tandem-Chromatographie: Zur chromatographischen Reinigung wird eine Säule aus 75 g DE-52 Zellulose (Whatman) mit 0,025 M Tris/HCl + 0,2 M NaCl, pH 7,5 äquilibriert und vor eine Affinitätssäule (60 ml) vorgeschaltet, die den monoklonalen Antikörper EBI-1 (siehe EP-A-0.119.476), gekuppelt an Sepharose 4B (mit Hilfe von BrCN-aktivierter Sepharose 4B, Pharmacia, hergestellt) enthält. Die Affinitätssäule enthält 480 mg des monoklonalen Antikörpers EBI-1, sie wird ebenfalls mit Tris/HCl + NaCl pH 7,5 wie oben beschrieben äquilibriert. Die Interferonlösung wird durch die beiden Säulen gepumpt und solange mit 0,025 M Tris/HCL + 0,2 M NaCl nachgewaschen, bis im Eluat nur mehr eine Extinktion $OD_{280}$nm von unter 0,1

gemessen wird. Danach wird die Vorsäule (DE-52 Zellulose) abgekuppelt, und die EBI-1 Säule solange weiter gewaschen, bis das Eluat proteinfrei ist ($OD_{280}$nm im Eluat unter 0,01). Das adsorbierte Interferon wird nunmehr mit Hilfe von 0,1 M Zitronensäure in 25%igem Äthylenglykol eluiert. Der Proteinpeak wird gesammelt.
Das saure Eluat der Antikörpersäule wird mit 2 N Ammoniak auf pH 4,5 eingestellt, und der entstandene Niederschlag abzentrifugiert.
Die letzte Reinigungstufe ist eine Ionenaustausch-Chromatographie an dem Träger MONO-S (Pharmacia). Eine Säule von 1 ml Bettvolumen (HR 5/5) wird an eine FPLC-Apparatur angeschlossen (Pharmacia) und in 0,1 M Na-Citrat pH 4,2 äquilibriert. Die IFN-Lösung wird aufgetragen, und das adsorbierte Interferon mit Hilfe eines pH-Gradienten eluiert (Puffer A: 0,1 M Na-Citrat pH 4,2 und Puffer B: 0,1 M Na-Phosphat pH 8,0). Das Interferon-omega1 wird bei einem pH von 7,0 eluiert und der IFN-Peak gesammelt (siehe Fig.6).

Übersicht über die Reinigung von IFN-omega1/α2(BglII)

Ausgangsmaterial: 145 g E.coli HB 101/pRH78r

| | Vol. (ml) | IFN[x)] (Einh.) | Protein[xx)] (mg) | Einh./mg | Ausb. (%) |
|---|---|---|---|---|---|
| Rohextrakt | 1470 | $16{,}9 \times 10^9$ | 3000 | $5{,}6 \times 10^6$ | 100 |
| nach Fällung Ammonsulfat u. Dialyse | 246 | $14{,}0 \times 10^9$ | 1970 | $7{,}1 \times 10^6$ | 83 |
| Eluat der Antikörpersäule | 11,1 | $10{,}3 \times 10^9$ | 12,9 | $800 \times 10^6$ | 61 |
| Überstand pH 4,5 | 11,9 | $7{,}4 \times 10^9$ | 9,3 | $800 \times 10^6$ | 44 |
| Pool nach MONO-S | 6,9 | $4{,}6 \times 10^9$ | 7,0 | $660 \times 10^6$ | 27 |

[x)] Die Bestimmung des Interferon-Gehalts erfolgt durch Messung der antiviralen Aktivität (CPE-Reduktionstest) mit Hilfe von A549-Zellen und Encephalomyocarditis-Virus (EMC-Virus). Als Standard dient IFN-α2(Arg).
[xx)] Die Proteinbestimmung erfolgt mit Hilfe des Bio-Rad Protein Assays. Der Standard war Serumalbumin.

Beurteilung der Reinigung

Das gereinigte Hybridinterferon-omega1/α2(BglI) ist homogen (siehe hierzu Fig. 7 - Gelpermeations-HPLC). Die erreichte spezifische Aktivität von etwa $800 \times 10^6$ Einheiten/mg Protein ist höher als die von IFN-α2(Arg) (ca. $300 \times 10^6$ Einheiten/mg Protein).

**Patentansprüche**

**1.** BglII-Hybridinterferone der allgemeinen Formel

```
R1 - Gln Ile Phe - R2
     CAG ATC TTC          (I)
      BglII
```

in der
BglII die gemeinsame BglII-Restriktionsstelle der α1-, α2-und omega-Interferone,
$R_1$ die Peptidsequenz eines α1- oder α2-Interferons, die durch die DNA-Sequenz dieser Interferone vor der BglII-Schnittstelle kodiert wird, und $R_2$ die Peptidsequenz eines omega-Interferons, die durch die DNA-Sequenz dieses Interferons nach der BglII-Schnittstelle kodiert wird, oder
$R_1$ die Peptidsequenz eines omega-Interferons, die durch die DNA-Sequenz dieses Interferons vor der BglII-Schnittstelle kodiert wird, und $R_2$ die Peptidsequenz eines α1- oder α2-Interferons, die durch die DNA-Sequenz dieser Interferone nach der BglII-Schnittstelle kodiert wird, bedeuten, deren N-terminale Met- oder N-Formyl-Met-Derivate und, falls die Peptidsequenz des Hybridinterferons eine Glykosylierungsstelle enthält, deren N-glykosylierte Derivate.

**2.** BgIII-Hybridinterferone gemäß Anspruch 1, dadurch gekennzeichnet, daß als α-Interferon das IFN-α2-(Arg) und als omega-Interferon das omega1(Glu)- oder omega1(Gly)-Interferon eingesetzt wird, deren N-terminale Met- oder N-Formyl-Met-Derivate und deren N-glykosylierte Derivate.

**3.** IFN-α2/omega1(BgIII)-Hybridinterferone gemäß Anspruch 2 der Formel

```
                    5                       10                      15
Cys Asp Leu Pro Gln Thr His Ser Leu Gly Ser Arg Arg Thr Leu

                    20                      25                      30
Met Leu Leu Ala Gln Met Arg Arg Ile Ser Leu Phe Ser Cys Leu

                    35                      40                      45
Lys Asp Arg Arg Asp Phe Gly Phe Pro Gln Glu Glu Phe Gly Asn

                    50                      55                      60
Gln Phe Gln Lys Ala Glu Thr Ile Pro Val Leu His Glu Met Ile

                    65                      70                      75
Gln Gln Ile Phe Ser Leu Phe His Thr Glu Arg Ser Ser Ala Ala

                    80                      85                      90
Trp Asn Met Thr Leu Leu Asp Gln Leu His Thr Gly Leu His Gln

                    95                      100                     105
Gln Leu Gln His Leu Glu Thr Cys Leu Leu Gln Val Val Gly Glu

                    110                     115                     120
Gly Glu Ser Ala -X- Ala Ile Ser Ser Pro Ala Leu Thr Leu Arg

                    125                     130                     135
Arg Tyr Phe Gln Gly Ile Arg Val Tyr Leu Lys Glu Lys Lys Tyr

                    140                     145                     150
Ser Asp Cys Ala Trp Glu Val Val Arg Met Glu Ile Met Lys Ser

                    155                     160                     165
Leu Phe Leu Ser Thr Asn Met Gln Glu Arg Leu Arg Ser Lys Asp

                    170
Arg Asp Leu Gly Ser Ser
```

in der
X für Gly oder Glu steht, deren N-terminale Met- oder N-Formyl-Met-Derivate und deren N-glykosylierte Derivate.

**4.** IFN-omega1/α2(BgIII) gemäß Anspruch 2 der Formel

```
                    5                       10                      15
Cys Asp Leu Pro Gln Asn His Gly Leu Leu Ser Arg Asn Thr Leu

                    20                      25                      30
Val Leu Leu His Gln Met Arg Arg Ile Ser Pro Phe Leu Cys Leu
```

| | | | | | | | | | | | | | | |
|---|---|---|---|---|---|---|---|---|---|---|---|---|---|---|
| | | | | 35 | | | | | 40 | | | | | 45 |
| Lys | Asp | Arg | Arg | Asp | Phe | Arg | Phe | Pro | Gln | Glu | Met | Val | Lys | Gly |
| | | | | 50 | | | | | 55 | | | | | 60 |
| Ser | Gln | Leu | Gln | Lys | Ala | His | Val | Met | Ser | Val | Leu | His | Glu | Met |
| | | | | 65 | | | | | 70 | | | | | 75 |
| Leu | Gln | Gln | Ile | Phe | Asn | Leu | Phe | Ser | Thr | Lys | Asp | Ser | Ser | Ala |
| | | | | 80 | | | | | 85 | | | | | 90 |
| Ala | Trp | Asp | Glu | Thr | Leu | Leu | Asp | Lys | Phe | Tyr | Thr | Glu | Leu | Tyr |
| | | | | 95 | | | | | 100 | | | | | 105 |
| Gln | Gln | Leu | Asn | Asp | Leu | Glu | Ala | Cys | Val | Ile | Gln | Gly | Val | Gly |
| | | | | 110 | | | | | 115 | | | | | 120 |
| Val | Thr | Glu | Thr | Pro | Leu | Met | Lys | Glu | Asp | Ser | Ile | Leu | Ala | Val |
| | | | | 125 | | | | | 130 | | | | | 135 |
| Arg | Lys | Tyr | Phe | Gln | Arg | Ile | Thr | Leu | Tyr | Leu | Lys | Glu | Lys | Lys |
| | | | | 140 | | | | | 145 | | | | | 150 |
| Tyr | Ser | Pro | Cys | Ala | Trp | Glu | Val | Val | Arg | Ala | Glu | Ile | Met | Arg |
| | | | | 155 | | | | | 160 | | | | | 165 |
| Ser | Phe | Ser | Leu | Ser | Thr | Asn | Leu | Gln | Glu | Ser | Leu | Arg | Ser | Lys |
| | | | | 170 | | | | | | | | | | |
| Glu | | | | | | | | | | | | | | |

und dessen N-terminales Met- oder N-Formyl-Met-Derivat.

**5.** Arzneimittel, enthaltend ein Hybridinterferon gemäß den Ansprüchen 1, 2 oder 4.

**6.** Verwendung eines Hybridinterferons gemäß den Ansprüchen 1, 2 oder 4 zur Herstellung eines Arzneimittels zur Behandlung von viralen Erkrankungen.

**7.** Rekombinantes DNA-Molekül, enthaltend eine kodierende Sequenz für die neuen Hybridinterferone gemäß den Ansprüchen 1 bis 4.

**8.** Vehikel kodierend für ein Hybridinterferon gemäß den Ansprüchen 1 bis 4, dadurch gekennzeichnet, daß dieses ein rekombinantes DNA-Molekül gemäß Anspruch 7 enthält, wobei vorzugsweise dieses zusätzlich die für die Expression erforderlichen Replikon- und Kontrollsequenzen und gegebenenfalls einen par-Lokus enthält.

**9.** Expressionsplasmid gemäß Anspruch 8, dadurch gekennzeichnet, daß die für die Expression erforderlichen Replikon- und Kontrollsequenzen aus dem Plasmid pER103 (DSM-Nr. 2773) stammen und die Formel

```
5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
                           |<----------Promotor--------------------

GCGAACCAGTTAACTAGTACACAAGTTCACGGCAACGGTAAGGAGGTTTAAGCT
CGCTTGGTCAATTGATCATGTGTTCAAGTGCCGTTGCCATTCCTCCAAATTCGA
---Promotor/Operator--*-mRNA-Start--------RBS-----*---Link-

TAAAGATGTGT------
ATTTCTACACACTAG--
-er--*---Gen------>
```

aufweisen, oder eine die Trp-Promotor-Aktivität erhaltende Modifikation.

**10.** Expressionsplasmide gemäß Anspruch 8, welche am Beispiel des Plasmids pRH 72 durch Fig. 4 charakerisiert sind und jeweils ca. 4,80 kb enthalten, dadurch gekennzeichnet, daß im Plasmid parpATER33, welches durch Fig. 1 charakterisiert ist und ca. 5,03 kb enthält, das große BglII/-SphI-Fragment mit dem kleinen BglII/SphI-Fragment des Plasmids pRHW13 oder pRHW14, welche durch Fig. 2 charakterisiert sind und jeweils ca. 4,80 kb enthalten, wobei pRHW13 für IFN-omega1(Glu) und pRHW14 für IFN-omega1(Gly) codiert, ligiert ist.

**11.** Expressionsplasmid pRH78r gemäß Anspruch 8, welches durch Fig. 5 charakterisiert ist und ca. 5,03 kb enthält, dadurch gekennzeichnet, daß in das mit BglII-linearisierte Plasmid pRH77, welches durch Fig. 5 charakterisiert ist, ca. 4,77 kb enthält und ausgehend vom Plasmid pRHW14 gemäß Fig. 2 erhalten wird, das 263 bp lange BglII-BglII-Fragment des Plasmids parpATER33 eingesetzt ist.

**12.** Transformierter Wirtsorganismus wie ein Prokaryot oder ein Eukaryot, vorzugsweise jedoch E.coli HB101, der die genetische Information gemäß Anspruch 7 enthält, wobei vorzugsweise die genetische Information in einem Vehikel, das die für die Replikation erforderlichen Replikon- und Kontrollsequenzen enthält, enthalten ist.

**13.** Bakterieller Wirt gemäß Anspruch 12, vorzugsweise E.coli HB101, transformiert mit einem Expressionsplasmid gemäß Anspruch 8, 9, 10 oder 11.

**14.** Verfahren zur Herstellung der DNA-Sequenzen gemäß Anspruch 7, dadurch gekennzeichnet, daß man diese durch Aufarbeiten eines Wirtsorganismus gemäß Anspruch 12 oder 13 erhält.

**15.** Verfahren zur Herstellung der Expressionsplasmide gemäß Anspruch 10, dadurch gekennzeichnet, daß das große Fragment, das man durch Verdauen des Plasmids parpATER33 mit BglII und SphI erhält, mit dem kleinen Fragment, das man durch Verdauen des Plasmids pRHW13 oder 14 mit BglII und SpI erhält, ligiert, und anschließend zur Replikation E.coli HB101 mit dem so erhaltenen Plasmid transformiert wird.

**16.** Verfahren zur Herstellung des Expressionsplasmids pRH78r gemäß Anspruch 11, dadurch gekennzeichnet, daß das Plasmid pRH77 mit BglII geschnitten und anschließend mit dem 263 bp langen Fragment, welches man durch Verdauen des Plasmids parpATER33 mit BglII erhält, ligiert und anschließend zur Replikation E.coli HB101 mit dem so erhaltenen Plasmid transformiert wird.

**17.** Verfahren zur Herstellung eines transformierten Wirtsorganismus gemäß Anspruch 12 oder 13, dadurch gekennzeichnet, daß ein Wirt, vorzugsweise ein bakterieller Wirt, mit einem Plasmid gemäß den Ansprüchen 8 bis 11 transformiert wird.

**18.** Verfahren zur Herstellung der Hybridinterferone gemäß den Ansprüchen 1 bis 4, dadurch gekennzeichnet, daß ein geeigneter bakterieller Wirtsorganismus mit genetischen Informationen, kodierend für diese Hybridinterferone transformiert wird, diese Informationen exprimiert werden und das so erhaltene Hybridinterferon aus diesem Wirtsorganismus isoliert wird.

**Claims**

**1.** BglII hybrid interferons of the general formula

$$\begin{array}{c} R_1 - \text{Gln Ile Phe} - R_2 \\ \text{C}\underline{\text{AG}}\ \underline{\text{ATC}}\ \underline{\text{T}}\text{TC} \\ \text{BglII} \end{array} \qquad \text{(I)}$$

wherein BglII represents the common BglII restriction site of $\alpha 1$, $\alpha 2$- and omega-interferons, $R_1$ represents the peptide sequence of an $\alpha 1$ or $\alpha 2$ interferon which is coded by the DNA sequence of these interferons in front of the BglII cutting site, and $R_2$ represents the peptide sequence of an omega-interferon which is coded by the DNA sequence of this interferon after the BglII cutting site, or $R_1$ represents the peptide sequence of an omega-interferon which is coded by the DNA sequence of this interferon before the BglII cutting site, and $R_2$ represents the peptide sequence of an $\alpha 1$- or $\alpha 2$-interferon which is coded by the DNA sequence of these interferons after the BglII cutting site, the N-terminal Met or N-formyl-Met derivatives thereof and, if the peptide sequence of the hybrid interferon contains a glycosylation site, the N-glycosylated derivatives thereof.

**2.** BglII hybrid interferons as claimed in claim 1, characterised in that IFN-$\alpha 2$(Arg) is used as the $\alpha$-interferon and omega1(Glu) or omega1(Gly) interferon is used as the omega interferon, the N-terminal Met or N-formyl-Met derivatives and N-glycosylated derivatives thereof.

**3.** IFN-$\alpha 2$/omega1(BglII) hybrid interferons as claimed in claim 2 of the formula

```
                    5                        10                       15
Cys Asp Leu Pro Gln Thr His Ser Leu Gly Ser Arg Arg Thr Leu

                    20                       25                       30
Met Leu Leu Ala Gln Met Arg Arg Ile Ser Leu Phe Ser Cys Leu

                    35                       40                       45
Lys Asp Arg Arg Asp Phe Gly Phe Pro Gln Glu Glu Phe Gly Asn

                    50                       55                       60
Gln Phe Gln Lys Ala Glu Thr Ile Pro Val Leu His Glu Met Ile

                    65                       70                       75
Gln Gln Ile Phe Ser Leu Phe His Thr Glu Arg Ser Ser Ala Ala

                    80                       85                       90
Trp Asn Met Thr Leu Leu Asp Gln Leu His Thr Gly Leu His Gln

                    95                       100                      105
Gln Leu Gln His Leu Glu Thr Cys Leu Leu Gln Val Val Gly Glu

                    110                      115                      120
Gly Glu Ser Ala -X- Ala Ile Ser Ser Pro Ala Leu Thr Leu Arg

                    125                      130                      135
Arg Tyr Phe Gln Gly Ile Arg Val Tyr Leu Lys Glu Lys Lys Tyr

                    140                      145                      150
Ser Asp Cys Ala Trp Glu Val Val Arg Met Glu Ile Met Lys Ser

                    155                      160                      165
Leu Phe Leu Ser Thr Asn Met Gln Glu Arg Leu Arg Ser Lys Asp

                    170
Arg Asp Leu Gly Ser Ser
```

wherein
X represents Gly or Glu, the N-terminal Met of N-formyl-Met derivatives and N-glycosylated derivatives thereof.

**4.** IFN-omega1/α2(BglII) as claimed in claim 2 of the formula

```
                    5                        10                       15
Cys Asp Leu Pro Gln Asn His Gly Leu Leu Ser Arg Asn Thr Leu

                    20                       25                       30
Val Leu Leu His Gln Met Arg Arg Ile Ser Pro Phe Leu Cys Leu
```

| | | | | | | | | | | | | | | |
|---|---|---|---|---|---|---|---|---|---|---|---|---|---|---|
| | | | | 35 | | | | | 40 | | | | | 45 |
| Lys | Asp | Arg | Arg | Asp | Phe | Arg | Phe | Pro | Gln | Glu | Met | Val | Lys | Gly |
| | | | | 50 | | | | | 55 | | | | | 60 |
| Ser | Gln | Leu | Gln | Lys | Ala | His | Val | Met | Ser | Val | Leu | His | Glu | Met |
| | | | | 65 | | | | | 70 | | | | | 75 |
| Leu | Gln | Gln | Ile | Phe | Asn | Leu | Phe | Ser | Thr | Lys | Asp | Ser | Ser | Ala |
| | | | | 80 | | | | | 85 | | | | | 90 |
| Ala | Trp | Asp | Glu | Thr | Leu | Leu | Asp | Lys | Phe | Tyr | Thr | Glu | Leu | Tyr |
| | | | | 95 | | | | | 100 | | | | | 105 |
| Gln | Gln | Leu | Asn | Asp | Leu | Glu | Ala | Cys | Val | Ile | Gln | Gly | Val | Gly |
| | | | | 110 | | | | | 115 | | | | | 120 |
| Val | Thr | Glu | Thr | Pro | Leu | Met | Lys | Glu | Asp | Ser | Ile | Leu | Ala | Val |
| | | | | 125 | | | | | 130 | | | | | 135 |
| Arg | Lys | Tyr | Phe | Gln | Arg | Ile | Thr | Leu | Tyr | Leu | Lys | Glu | Lys | Lys |
| | | | | 140 | | | | | 145 | | | | | 150 |
| Tyr | Ser | Pro | Cys | Ala | Trp | Glu | Val | Val | Arg | Ala | Glu | Ile | Met | Arg |
| | | | | 155 | | | | | 160 | | | | | 165 |
| Ser | Phe | Ser | Leu | Ser | Thr | Asn | Leu | Gln | Glu | Ser | Leu | Arg | Ser | Lys |
| | | | | 170 | | | | | | | | | | |
| Glu | | | | | | | | | | | | | | |

and the N-terminal Met or N-formyl-Met derivative thereof.

5. Pharmaceutical composition containing a hybrid interferon as claimed in claims 1, 2 or 4.

6. Use of a hybrid interferon as claimed in claim 1, 2 or 4 for preparing a pharmaceutical composition for the treatment of viral diseases.

7. Recombinant DNA molecule containing a coding sequence for the new hybrid interferons as claimed in claims 1 to 4.

8. Vector coding for a hybrid interferon as claimed in claims 1 to 4, characterised in that it contains a recombinant DNA molecule as claimed in claim 7, whilst preferably it additionally contains the replicon and control sequences required for expression and optionally a par-locus.

9. Expression plasmid as claimed in claim 8, characterised in that the replicon and control sequences required for the expression originate from the plasmid pER103 (DSM No. 2773) and have the formula

```
5'AATTCACGCTGATCGCTAAAACATTGTGCAAAAAGAGGGTTGACTTTGCCTTC
3'     GTGCGACTAGCGATTTTGTAACACGTTTTTCTCCCAACTGAAACGGAAG
                         |<--------Promoter-------------------

GCGAACCAGTTAACTAGTACACAAGTTCACGGCAACGGTAAGGAGGTTTAAGCT
CGCTTGGTCAATTGATCATGTGTTCAAGTGCCGTTGCCATTCCTCCAAATTCGA
---Promoter/Operator->*<-mRNA-Start--------RBS---->*<--Link-

TAAAGATGTGT------
ATTTCTACACACTAG--
-er->*<--Gene---->
```

or a modification thereof which retains trp promoter activity.

**10.** Expression plasmids according to claim 8 which are characterised by the example of the plasmid pRH 72 according to Fig. 4 and each contain about 4.80 kb, characterised in that, in the plasmid parpATER33, which is characterised by Fig. 1 and contains about 5.03 kb, the large BglII/SphI fragment is ligated with the small BglII/SphI fragment of the plasmid pRHW13 or pRHW14, which are characterised by Fig. 2 and each contain about 4.80 kb, whilst pRHW13 codes for IFN-omega1(Glu) and pRHW1414 codes for IFN-omega1(Gly).

**11.** Expression plasmid pRH78r according to claim 8 which is characterised by Fig. 5 and contains about 5.03 kb, characterised in that the BglII-BglII fragment of the plasmid parpATER 33 which is 263 bp long is inserted in the plasmid pRH77 linearised with BglII characterised by Fig. 5, which contains about 4.77 kb and is obtained from the plasmid pRHW14 according to Fig. 2.

**12.** Transformed host organism such as a prokaryote or eukaryote, preferably E. coli HB101, which contains the genetic information according to claim 7, whilst preferably the genetic information is contained in a vector which contains the replicon and control sequences required for replication.

**13.** Bacterial host according to claim 12, preferably E. coli HB101, transformed with an expression plasmid according to claim 8, 9, 10 or 11.

**14.** Process for preparing the DNA sequences as claimed in claim 7, characterised in that they are obtained by working up a host organism as claimed in claim 12 or 13.

**15.** Process for preparing the expression plasmids as claimed in claim 10, characterised in that the large fragment which is obtained by digesting the plasmid parpATER33 with BglII and SphI, is ligated to the small fragment which is obtained by digesting the plasmid pRHW13 or pRHW14 with BglII and SphI, and subsequently, for replication, E. coli HB101 is transformed with the plasmid thus obtained.

**16.** Process for preparing the expression plasmid pRH78r as claimed in claim 11, characterised in that the plasmid pRH77 is cut with BglII and subsequently ligated to the 263 bp long fragment which is obtained by digesting the plasmid parpATER33 with BglII and SphI, and subsequently, for replication, E. coli HB101 is transformed with the plasmid thus obtained.

**17.** Process for preparing a transformed host organism as claimed in claim 12 or 13, characterised in that a host, preferably a bacterial host, is transformed with a plasmid as claimed in claims 8 to 11.

**18.** Process for preparing the hybrid interferons as claimed in claims 1 to 4, characterised in that a suitable bacterial host organism is transformed with genetic information coding for these hybrid interferons, this

information is expressed and the resulting hybrid interferon is isolated from the host organism.

**Revendications**

1. Interférons hybrides BglII de formule générale

$$\begin{array}{c} R_1 \text{ - Gln Ile Phe - } R_2 \\ \text{CAG ATC TTC} \\ \text{BglII} \end{array} \qquad (I)$$

dans laquelle
BglII représente le site de restriction BglII commun des interférons α1, α2 et oméga,
$R_1$ représente la séquence peptidique d'un interféron α1 ou α2 qui est codée par la séquence d'ADN de ces interférons en amont du site de coupure BglII, et $R_2$ représente la séquence peptidique d'un interféron oméga qui est codée par la séquence d'ADN de cet interféron en aval du site de coupure BglII, ou bien
$R_1$ représente la séquence peptidique d'un interféron oméga qui est codée par la séquence d'ADN de cet interféron en amont du site de coupure BglII, et $R_2$ représente la séquence peptidique d'un interféron α1 ou α2 qui est codée par la séquence d'ADN de ces interférons en aval du site de coupure BglII, leurs dérivés Met ou N-formyl-Met N-terminaux et, lorsque la séquence peptidique de l'interféron hybride contient un site de glycosylation, leurs dérivés N-glycosylés.

2. Interférons hybrides BglII selon la revendication 1, caractérisés en ce que l'on utilise comme interféron α le IFN-α2(Arg) et comme interféron oméga l'interféron oméga1(Glu) ou oméga1(Gly), leurs dérivés Met ou N-formyl-Met N-terminaux et leurs dérivés N-glycosylés.

3. Interférons hybrides IFN-α2/oméga1(BglII) selon la revendication 2, de formule

5 10 15

Cys Asp Leu Pro Gln Thr His Ser Leu Gly Ser Arg Arg Thr Leu

20 25 30

Met Leu Leu Ala Gln Met Arg Arg Ile Ser Leu Phe Ser Cys Leu

35 40 45

Lys Asp Arg Arg Asp Phe Gly Phe Pro Gln Glu Glu Phe Gly Asn

50 55 60

Gln Phe Gln Lys Ala Glu Thr Ile Pro Val Leu His Glu Met Ile

65 70 75

Gln Gln Ile Phe Ser Leu Phe His Thr Glu Arg Ser Ser Ala Ala

80 85 90

Trp Asn Met Thr Leu Leu Asp Gln Leu His Thr Gly Leu His Gln

95 100 105

Gln Leu Gln His Leu Glu Thr Cys Leu Leu Gln Val Val Gly Glu

110 115 120

Gly Glu Ser Ala -X- Ala Ile Ser Ser Pro Ala Leu Thr Leu Arg

125 130 135

Arg Tyr Phe Gln Gly Ile Arg Val Tyr Leu Lys Glu Lys Lys Tyr

140 145 150

Ser Asp Cys Ala Trp Glu Val Val Arg Met Glu Ile Met Lys Ser

155 160 165

Leu Phe Leu Ser Thr Asn Met Gln Glu Arg Leu Arg Ser Lys Asp

170

Arg Asp Leu Gly Ser Ser

dans laquelle

X représente Gly ou Glu, leurs dérivés Met ou N-formyl-Met N-terminaux et leurs dérivés N-glycosylés.

**4.** IFN-oméga1/α2(BglII) selon la revendication 2 de formule

```
                    5                  10                  15
Cys Asp Leu Pro Gln Asn His Gly Leu Leu Ser Arg Asn Thr Leu

                   20                  25                  30
Val Leu Leu His Gln Met Arg Arg Ile Ser Pro Phe Leu Cys Leu

                   35                  40                  45
Lys Asp Arg Arg Asp Phe Arg Phe Pro Gln Glu Met Val Lys Gly

                   50                  55                  60
Ser Gln Leu Gln Lys Ala His Val Met Ser Val Leu His Glu Met

                   65                  70                  75
Leu Gln Gln Ile Phe Asn Leu Phe Ser Thr Lys Asp Ser Ser Ala

                   80                  85                  90
Ala Trp Asp Glu Thr Leu Leu Asp Lys Phe Tyr Thr Glu Leu Tyr

                   95                 100                 105
Gln Gln Leu Asn Asp Leu Glu Ala Cys Val Ile Gln Gly Val Gly

                  110                 115                 120
Val Thr Glu Thr Pro Leu Met Lys Glu Asp Ser Ile Leu Ala Val
```

```
                    125                 130                 135
Arg Lys Tyr Phe Gln Arg Ile Thr Leu Tyr Leu Lys Glu Lys Lys

                    140                 145                 150
Tyr Ser Pro Cys Ala Trp Glu Val Val Arg Ala Glu Ile Met Arg

                    155                 160                 165
Ser Phe Ser Leu Ser Thr Asn Leu Gln Glu Ser Leu Arg Ser Lys

                    170
Glu
```

et son dérivé Met ou N-formyl-Met N-terminal.

5. Médicament contenant un interféron hybride selon la revendication 1, 2 ou 4.

6. Utilisation d'un interféron hybride selon la revendication 1, 2 ou 4 pour la préparation d'un médicament pour le traitement des maladies virales.

7. Molécule d'ADN recombiné contenant une séquence codant pour les nouveaux interférons hybrides selon les revendications 1 à 4.

8. Véhicule codant pour un interféron hybride selon les revendications 1 à 4, caractérisé en ce qu'il contient une molécule d'ADN recombiné selon la revendication 7, et contenant de préférence en outre les séquences de réplicon et de contrôle nécessaires pour l'expression et éventuellement un locus par.

9. Plasmide d'expression selon la revendication 8, caractérisé en ce que les séquences de réplicon et de contrôle nécessaires pour l'expression proviennent du plasmide pER103 (DSM N°. 2773) et présentent la formule

```
5'AATTCACGCTGATCGCTAAAACATTGTGCAAAAAGAGGGTTGACTTTGCCTTC
3'     GTGCGACTAGCGATTTTGTAACACGTTTTTCTCCCAACTGAAACGGAAG
                 <-----------promoteur------------------

GCGAACCAGTTAACTAGTACACAAGTTCACGGCAACGGTAAGGAGGTTTAAGCT
CGCTTGGTCAATTGATCATGTGTTCAAGTGCCGTTGCCATTCCTCCAAATTCGA
---promoteur/opérateur-*initiation ARNm----RBS-*-----li-

TAAAGATGTGT------
ATTTCTACACACTAG--
eur-*-gène--->
```

ou une modification présentant l'activité promoteur Trp.

**10.** Plasmides d'expression selon la revendication 8, qui sont caractérisés comme le plasmide pRH 72 par la figure 4 et qui contiennent chacun environ 4,80 kb, caractérisés en ce que, dans le plasmide parpATER33, qui est caractérisé par la figure 1 et qui contient environ 5,03 kb, le grand fragment BglII/SphI est ligaturé avec le petit fragment BglII/SphI du plasmide pRHW13 ou pRHW14, qui sont caractérisés par la figure 2 et qui contiennent chacun environ 4,80 kb, pRHW13 codant pour IFN-oméga1(Glu) et pRHW14 codant pour IFN-oméga1(Gly).

**11.** Plasmide d'expression pRH78r selon la revendication 8, qui est caractérisé par la figure 5 et qui contient environ 5,03 kb, caractérisé en ce que le fragment BglII-BglII long de 263 pb du plasmide parpATER33 est inséré dans le plasmide pRH77 linéarisé avec BglII, qui est caractérisé par la figure 5, qui contient environ 4,77 kb et qui est obtenu à partir du plasmide pRHW14 selon la figure 2.

**12.** Organisme hôte transformé tel qu'un procaryote ou un eucaryote, mais de préférence E. coli HB101, qui contient l'information génétique selon la revendication 7, l'information génétique étant de préférence contenue dans un véhicule qui contient les séquences de réplicon et de contrôle nécessaires pour la réplication.

**13.** Hôte bactérien selon la revendication 12, de préférence E. coli HB101, transformé avec un plasmide d'expression selon la revendication 8, 9, 10 ou 11.

**14.** Procédé de préparation des séquences d'ADN selon la revendication 7, caractérisé en ce qu'on les obtient par traitement d'un organisme hôte selon la revendication 12 ou 13.

**15.** Procédé d'obtention des plasmides d'expression selon la revendication 10, caractérisé en ce que le grand fragment que l'on obtient par digestion du plasmide parpATER33 avec BglII et SphI est ligaturé avec le petit fragment que l'on obtient par digestion du plasmide pRHW13 ou 14 avec BglII et SpI, puis E. coli HB101 est transformé avec le plasmide ainsi obtenu pour la réplication.

**16.** Procédé d'obtention du plasmide d'expression pRH78r selon la revendication 11, caractérisé en ce que le plasmide pRH77 est coupé avec BglII puis est ligaturé avec le fragment de 263 pb que l'on obtient par digestion du plasmide parpATER33 avec BglII, puis E. coli HB101 est transformé avec le plasmide ainsi obtenu pour la réplication.

**17.** Procédé d'obtention d'un organisme hôte transformé selon la revendication 12 ou 13, caractérisé en ce qu'un hôte, de préférence un hôte bactérien, est transformé avec un plasmide selon les revendications 8 à 11.

**18.** Procédé de préparation des interférons hybrides selon les revendications 1 à 4, caractérisé en ce qu'un organisme hôte bactérien approprié est transformé avec des informations génétiques codant pour ces interférons hybrides, ces informations sont exprimées et l'interféron hybride ainsi obtenu est isolé à partir de cet organisme hôte.

Fig. 1

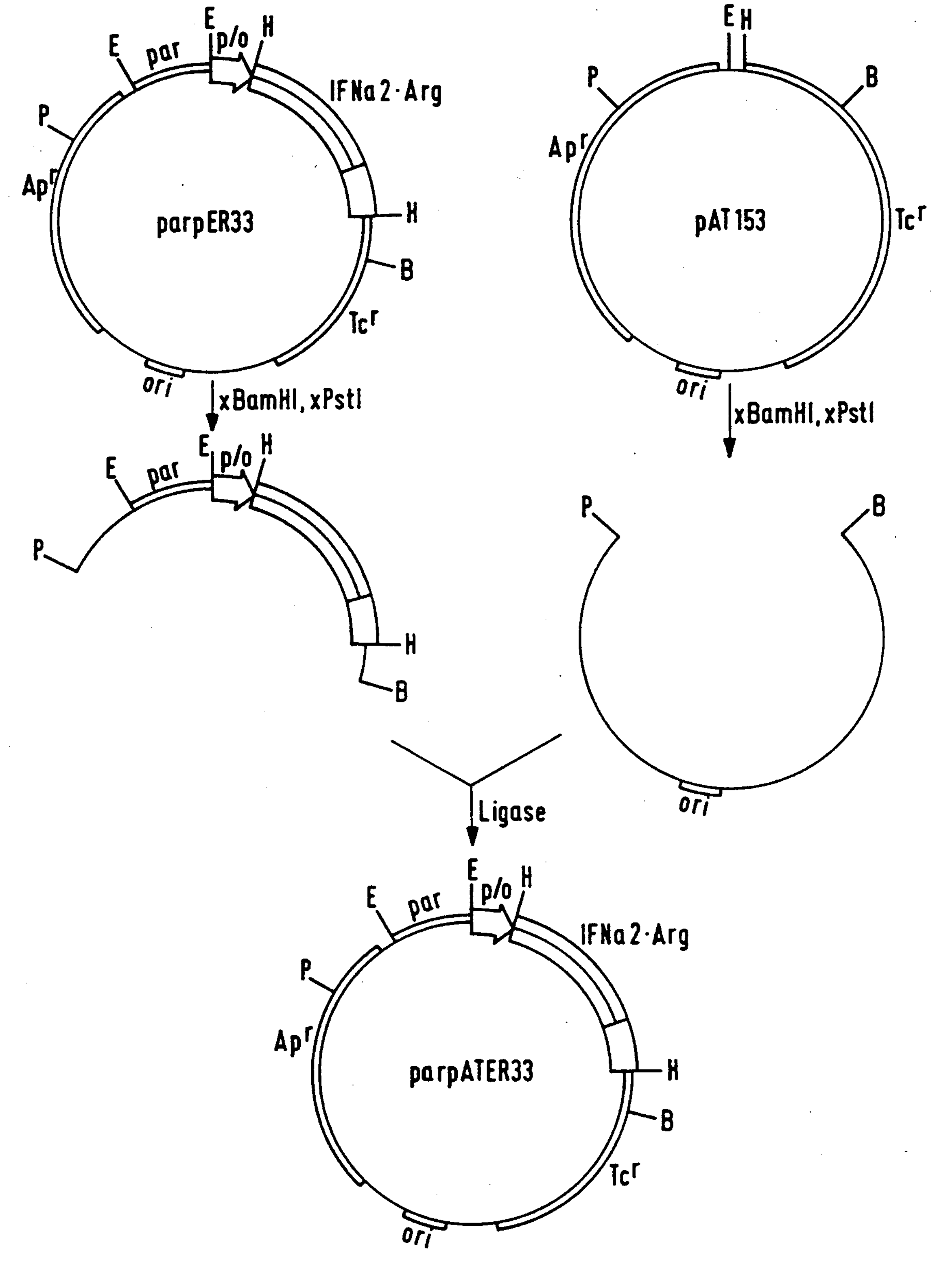
E
par
E
p/o
H
IFNa 2·Arg
P
Apr
parpER33
H
B
Tcr
ori
xBamHI, xPstI
E
H
P
B
Apr
pAT 153
Tcr
ori
xBamHI, xPstI
E
par
E
p/o
H
P
H
B
P
B
ori
Ligase
E
par
E
p/o
H
IFNa 2·Arg
P
Apr
parpATER33
H
B
Tcr
ori

Fig. 2

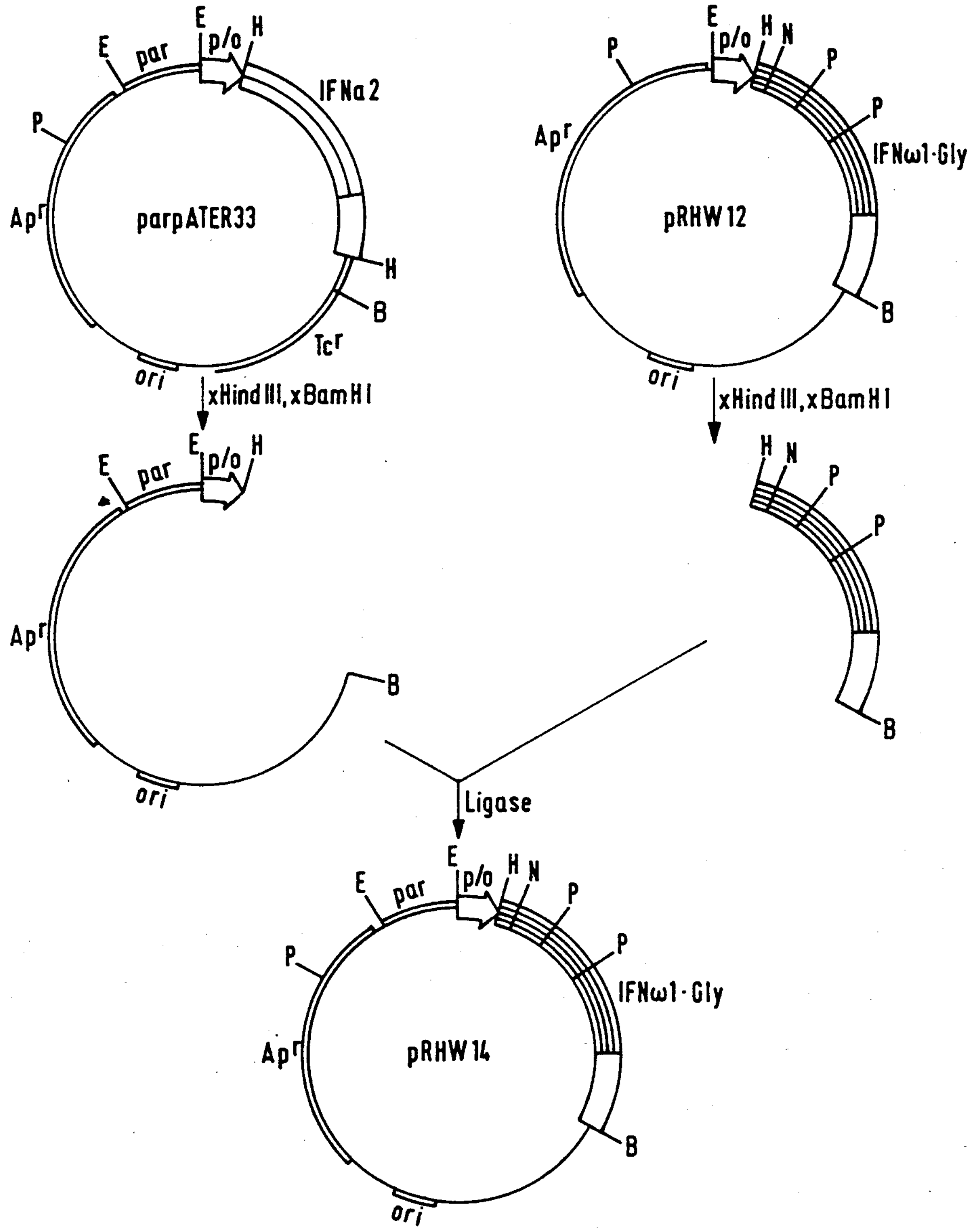
E
par
E
p/o
H
IFNa2
P
Apr
parpATER33
H
B
Tcr
ori
xHind III, xBamH I
E
p/o
H
N
P
P
Apr
IFNω1-Gly
pRHW12
B
ori
xHind III, xBamH I
E
par
E
p/o
H
Apr
B
ori
H
N
P
P
B
Ligase
E
par
E
p/o
H
N
P
P
P
IFNω1-Gly
Apr
pRHW14
B
ori

Fig. 3

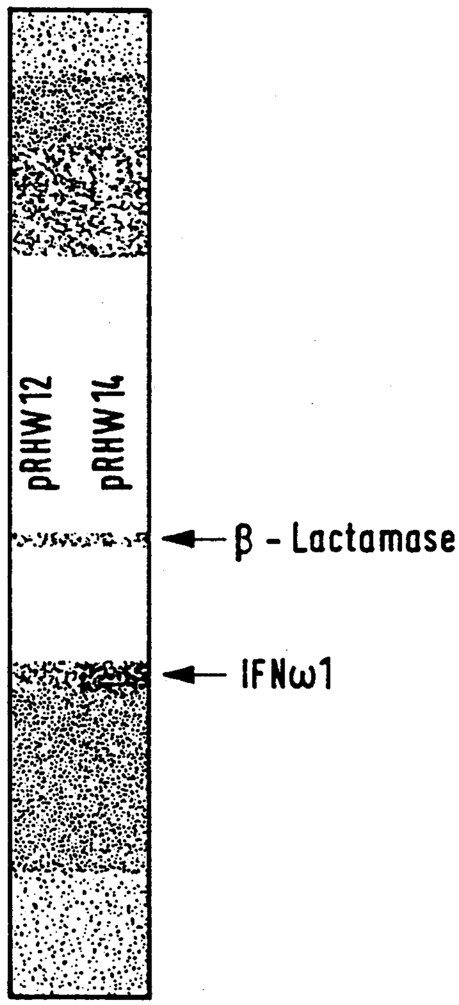

Fig. 4

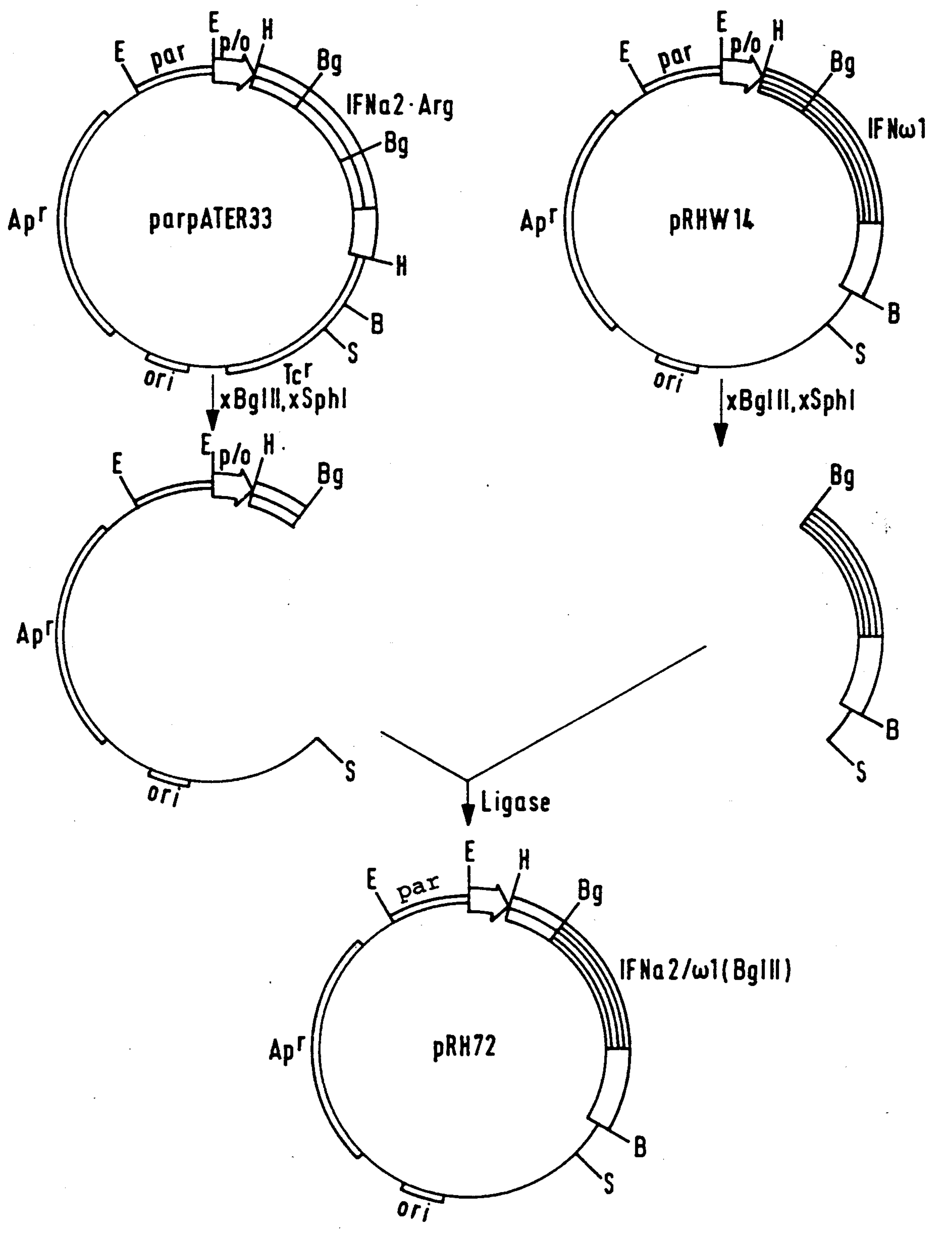
E
par
E
p/o
H
Bg
IFNa2·Arg
Bg
Ap^r
parpATER33
H
B
S
ori
Tc^r
xBglII,xSphI
E
par
E
p/o
H
Bg
IFNω1
Ap^r
pRHW14
B
S
ori
xBglII,xSphI
E
E
p/o
H
Bg
Ap^r
S
ori
Bg
B
S
Ligase
E
par
E
H
Bg
IFNa2/ω1(BglII)
Ap^r
pRH72
B
S
ori

Fig. 5

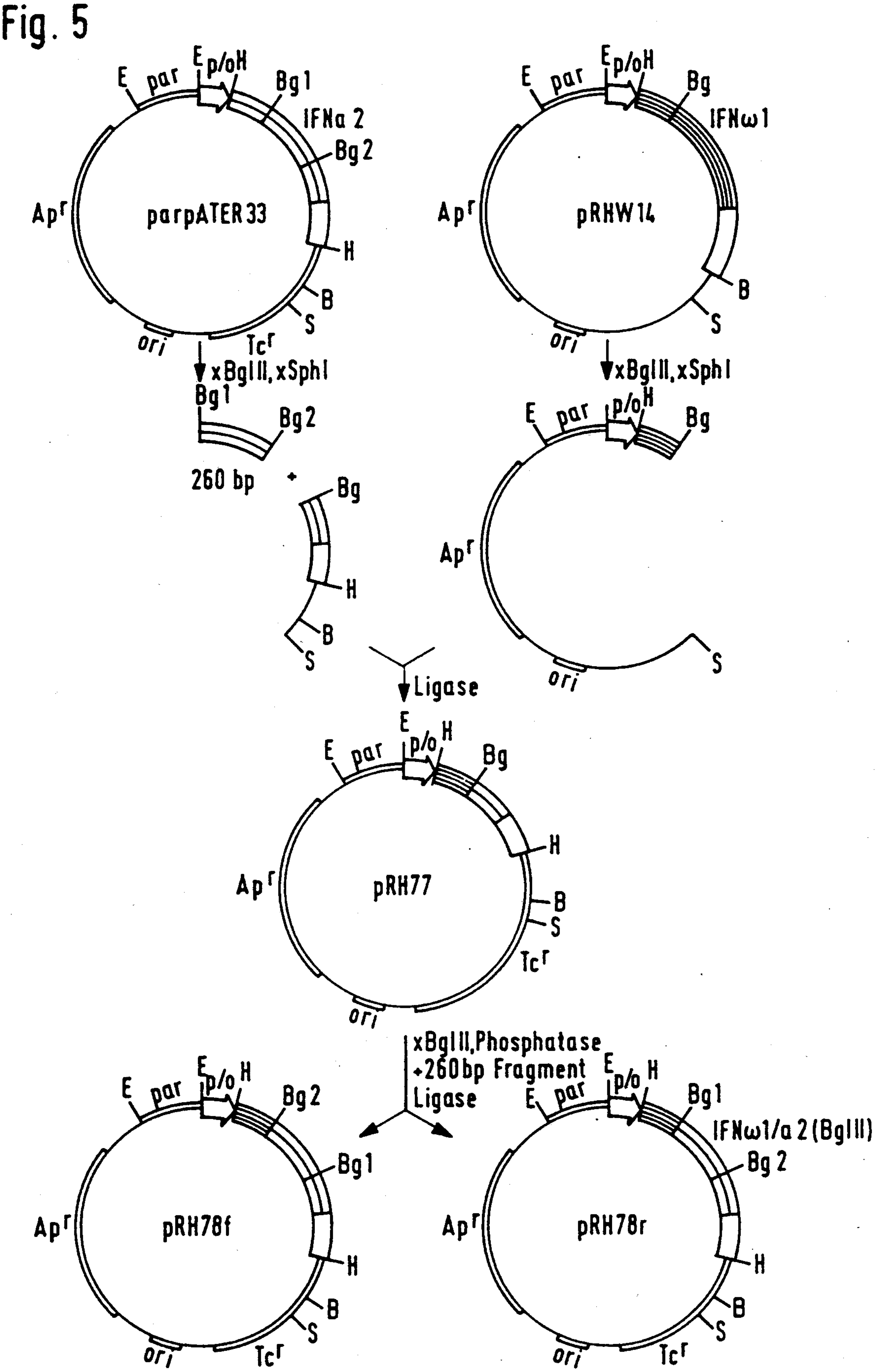
E
par
E
p/o
H
Bg1
IFNα 2
Bg2
Ap^r
parpATER33
H
B
S
ori
Tc^r
xBglII, xSphI
pRHW14
IFNω1
Bg
260 bp
+
Ligase
pRH77
xBglII, Phosphatase
+260bp Fragment
Ligase
pRH78f
pRH78r
IFNω1/α2 (BglII)

Fig. 6

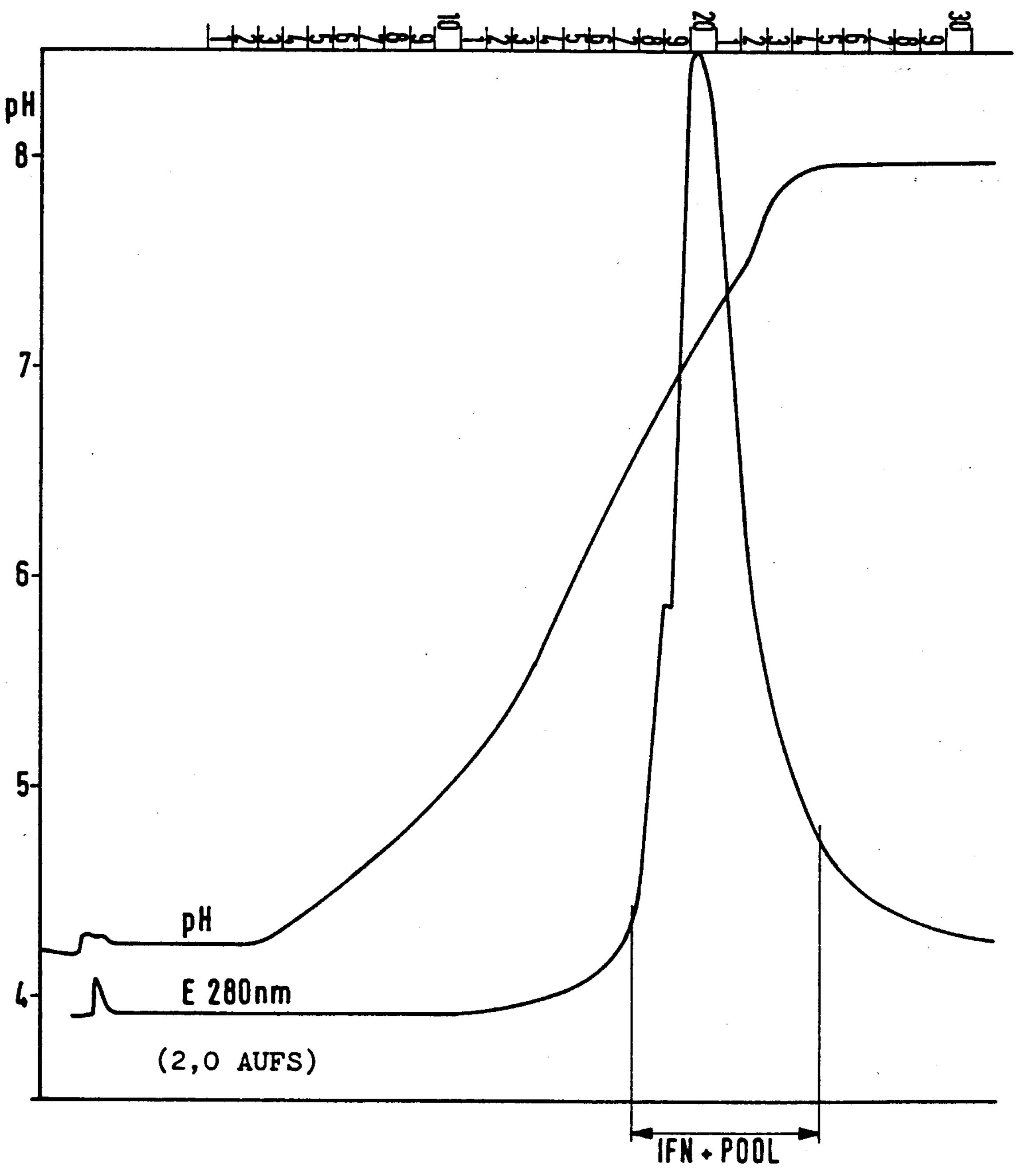

Fig. 7

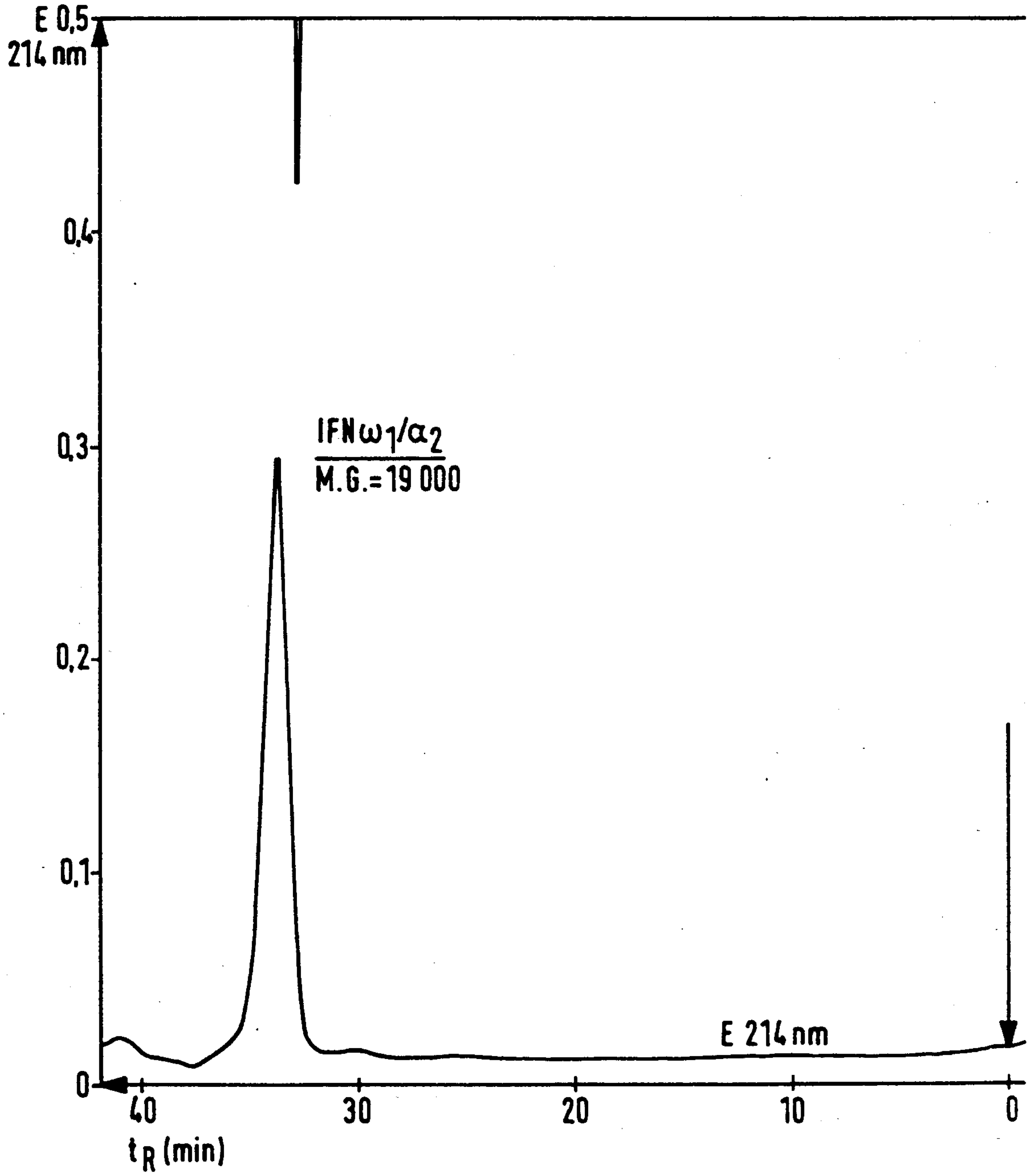
E 0,5
214 nm
0,4
0,3
0,2
0,1
0
IFNω1/α2
M.G.=19 000
E 214 nm
40
30
20
10
0
tR (min)